# EUROPEAN PATENT APPLICATION

(11) **EP 1 995 312 A1**
(43) Date of publication of application: **26.11.2008**
(21) Application number: 07108728.2
(22) Date of filing: 23.05.2007
(51) Int. Cl.: C12N 9/10, C12N 15/82, C12P 21/00, A01H 5/00

(54) **Alg3 mutant**

(71) Applicant: Plant Research International B.V., 6708 PB Wageningen (NL)
(72) Inventor: Bosch, Hendrik, Jan, 6708 NA Wageningen (NL); van der Krol, Alexander, Ronald, 3512 JR Utrecht (NL)
(74) Representative: van Loon, C.J.J.

(57) **Abstract**

The present invention relates to a method to produce proteins reduced in high mannose type glycans in a plant or plant cell by partially inhibiting the expression of the Alg3 gene in said plant or plant cell. Said partial inhibition can be provided for by inserting the Alg3 mutant gene from Arabidopsis as derived from the plant line Salk_040296c or by partially inhibiting the endogenous Alg3 gene by providing the plant or plant cell either with an antisense expression, a sense co-suppressing or with an RNA inhibition genetic construct. Optionally also tissue-specific expression of said inhibiting constructs can yield partial inhibition in a plant.

## Description

The invention relates to the field of plant biotechnological engineering, especially to production of glycoproteins, more particularly to altered glycosylation patterns on glycoproteins.

### Background

In all eukaryotes studied so far, N-glycans biosynthesis starts with the assembly of a lipid bound (Glc₃Man₉GlcNAc₂-PP-dol) precursor oligosaccharide. The assembly of this precursor starts at the cytoplasmic side of the endoplasmatic reticulum (ER) where two N-acetylglucosamine-phosphates and five mannose residues are transferred to dolichol-phosphate giving rise to Man₅GlcNAc₂-PP-Dol intermediate. Then, in a process not well understood yet, the Man₅GlcNAc₂-PP-Dol flips to the luminal side of the ER where the residual four mannose and three glucose residues are stepwise added by distinctive glycosyltransferases. (Snider, M.D. et al., 1980, Cell 21:385-392; Helenius, J. et al., 2002, Nature 415:447-450). This lipid linked Glc₃Man₉GlcNAc2- moiety is then transferred en bloc by the multisubunit oligosaccharyltransferase complex (OST) to the Y-amido group of selected asparagines of nascent proteins which enter the ER (Kornfeld, R. and Kornfeld, S., 1985, Annu. Rev. Biochem. 54:631-664). Through a series of trimming reactions by exoglycosidases in the ER and cis-Golgi compartments, the so-called "high mannose" (Man₉GlcNAc₂ to Man₅GlcNAc₂) glycans are formed. First, the glucose residues are removed from the glycan in a process which controls the folding and quality of newly synthesized glycoproteins (Roth et al, 2003. Until the glycoprotein is correctly folded, the protein cycles between a deglucosylated and reglucosylated state by the concerted action of glycosidases, calnexin, calreticulin and UPD-Glc: glycoprotein glucosyltransferase which interacts with malfolded proteins. Accumulation of malfolded proteins in the ER triggers the unfolded protein response (UPR) through which, amongst others, the expression of enzymes and chaperones like disulfide isomerase (PDI), binding protein (BiP) and calnexin and calreticulin are induced. After removal of the three glucose residues and all four α-(1,2) linked mannose residues, the formation of complex type glycans starts in the Golgi with the transfer of the first GlcNAc onto Man₅GlcNAc₂ and continues through the action of glycosidases and glycosyltransferases while the glycoprotein is progressing through the secretory pathway. In plants, complex glycans are characterised by the presence of β(1,2)-xylose residues linked to the Man-3 and/or an α(1,3)-fucose residue linked to GlcNAc-1 (Lerouge et al., 1998*). Exoglycosidases are known to rapidly trim glycoproteins stored in plant vacuoles leading to truncated Man₃XylFucGlcNAc₂ type complex glycans (Vitale and Chrispeels, 1984*; Lerouge et al., 1998*). Since any of the above described reactions may not go to completion, an enormous heterogeneity in N-glycan structures, including high mannose precursors, can be found on the endogeneous glycoprotein pool, but also even on a single polypeptide (Sturm et al., 1987*, Elbers et.al., 2001*).

To date only few Arabidopsis genes involved in N-glycan assembly or N-glycan processing in the ER has been described in literature. Three orthologues of OST subunit have been identified in Arabidopsis; DAD1 (Grallois et al, 1997), STT3 (Koiwa et al, 2003) and DGL1, the orthologue of WBP1/OST48 (Lerouxel et al, 2005) and also knf/gcsI (Boisson et al, 2001; Gillmor et al, 2002) and rsw3 (Burn et al, 2002) respectively altered in the *Arabidopsis* α-glucosiclase I and II. The *stt3a*/*stt3b* double mutant (Koiwa et al, 2003) and the *dgl1-2* mutant are lethal at early stages of embryo development whereas *knf*/*gcsI* is described as late embryo-lethal mutants. All these mutants show a moderate to severe underglycosylation defect. So plants mutated early in N-glycan biosynthesis are also earlier disturbed during development.

*Saccharomyces cerevisiae* is often used as a model for the study of eukaryotic N-glycan processing and many mutants defective in the biosynthesis of the Glc₃Man₉GlcNAc₂-PP-Dol have been isolated which has lead to the identification and characterisation of numerous ALG (Asparagine Linked Glycosylation) genes. Most of the *alg* mutations in *S. cerevisiae* do not result in a growth phenotype, but in combination with a conditional defect in the oligosaccharyltransferase activity a synthetic phenotype may arise (Staglijar et al., 1994*) that allows the identification of the corresponding genes and to define the pathway. It seems that the substrate specificity of the individual glycosyltransferases ensures an ordered assembly of the oligosaccharide (Burda et al., 1999*). The enzyme Dol-P-Man:Man₅GlcNAc₂-PP-Dol mannosyltransferase is responsible for the first Dol-P-Man-dependent mannosylation step at the luminal side of the ER, converting Man₅GlcNAC₂-PP-Dol to Man₆GlcNAc₂-PP-Dol (Sharma et al., 2001*). This enzyme was first cloned and characterized in *S. cerevisaae* and named *alg3.* In the *alg3* mutant Man₅GlcNAc₂-PP-Dol accumulates (Huffaker, T.C. and Robbins, P.W., 1983, Proc. Natl. Acad. Sci. USA 80:7466-7470), which is transferred to the nascent polypeptide chain, but results in an underglycosylation of secretory glycoproteins (Huffaker and Robbins 1983; Verostek, M.F. et al., 1993, J. Biol. Chem. 268:12095-12103; Verostek, M.F. et al, 1993 b*; Zufferey, R. et al., 1995, EMBO J. 14:4949-4960). Homologues of the yeast ALG genes have been identified in *P.pastoris* and *S. pombe,* but also in the fruit fly *D. melanogaster* and mammals (Huffacker and Robbins, 1982*, 1983; Runge et al., 1984*; Runge and Robbins, 1986*; Davidson et al, 2004*). Relatively little is known about lipid linked N-glycosylation in plants and no plant ALG genes have been characterised yet.

Although the advantages of plants as factories of proteinaceous substances are explained mostly in the light of biopharmaceuticals, plants are also useful for production of other proteins, e.g. industrial enzymes and the like, because of their capability of glycosylation leading e. g. to higher stability. Today, the utilization of plants for the production heterologous (glyco)proteins for therapeutic and other use is investigated in plants such as soy, tobacco, potato, rice, mosses, duckweed, potato, corn and rapeseed as well as from plant cell cultures, and the (glyco)proteins produced therein include monoclonal antibodies, hormones, vaccine antigens, enzymes and blood proteins. Some of these proteins have already proven their efficacy in human volunteers.

A drawback of glycoprotein production in plants relates to the glycosylation pattern of the glycoproteins produced in plants. Like other heterologous expression systems, plants exhibit a different glycosylation profile compared to mammals. In contrast to bacteria, having no N-linked glycans, and yeast, having only *N*-linked glycans of the high mannose type, plants are, as discussed above, able to produce proteins with *N*-linked glycans of the complex type. However, plant glycoproteins have complex *N*-linked glycans not found in mammals. An additional issue is the fact that many expression systems, including plant based expression systems such as, but not limited to tobacco (Elbers et.al. 2001), produce glycoproteins with high mannose type glycans. The presence of these high mannose type glycoproteins may not only have undesired properties by themselves, but also reduce the homogeneity of the glycoprotein product.

### Summary of the invention

The current invention now describes a method to modify glycosylation in plant cells or plants by partially inhibiting Alg3 expression, specifically, wherein said modification concerns N-glycan biosynthesis, more specifically wherein said modification comprises a reduction of glycoproteins with high mannose type glycans.

The method of the invention is preferably practiced by introducing a mutant Alg3 gene in the plant cell or plant, preferably wherein the Alg3 gene is an Arabidopsis Alg3 gene, more preferably wherein the mutant Alg3 gene has a DNA insert between intron 11 and exon 12. Preferably said mutant Alg3 gene is derived from the Arabidopsis plant line Salk_040296c.

Also part of the invention is a splice mutant of the Arabidopsis Alg3 gene, characterised in that it has a DNA insert between intron 11 and exon 12, preferably wherein said splice mutant is derived from the Arabidopsis plant line Salk_040296c. Of course the wild-type Salk_040296c plant is not included in the scope of this invention. Another embodiment of the present invention is the use of a splice mutant according to the invention for the reduction of high mannose type glycans in plant cells or plants. Therefore, also part of the invention is a transgenic plant cell or plant in which the expression of Alg3 is partially inhibited, preferably a plant which comprises a splice mutant according to the invention. In another embodiment such a transgenic plant comprises an antisense Alg3 construct, optionally under control of a tissue-specific promoter. Alternatively, such a transgenic plant comprises an Alg3 dsRNAi construct under control of a tissue specific promoter. A further alternative is a transgenic plant which comprises a co-sense Alg3 construct, optionally under control of a tissue-specific promoter. Preferably, in the above mentioned transgenic plants the tissue-specific promoter is able to drive expression in tissues selected from the group consisting of storage organs (such as tubers or seeds), leaves and roots.

Further part of the invention is a method to produce glycoproteins which are reduced in high mannose type glycans in plants by culturing a transgenic plant according to the invention, optionally providing said plant with a nucleic acid encoding a protein of interest, and harvesting the glycosylated proteins.

### Legends to the figures

Figure 1: Proposed wild-type (A) and Alg3 mutant pathway (B) of N-glycosylation in *Arabidopsis thaliana.*
Figure 2. Molecular Analysis. Schematic representation of the AtAlg3 gene organisation The structure of the gene is deduced from the comparison between the genomic and cDNA sequences. The insertion site from the left border of the T-DNA in AtAlg3-1 and AtAlg3-2 mutants is determined by PCR and sequencing.
Figure 3. Alignment of the alg3 protein sequences derived from *Saccharomyces cerevisiae* (AAA75352), Human (CR616285) and *Arabidopsis thaliana* (AAC63631) using ClustalW (http://www.ch.embnet.org/software/ClustalW.html). Identical and similar residues are shaded black and grey respectively, putative N-glycosylation sites of AtAlg3 are underlined.
Figure 4: Expression of AtAlg3 in AtAlg3-2 mutant. PCR on wildtype and AtAlg3-2 cDNA using primers flanking the putative splice site.
Figure 5: Expression of AtAlg3 in wild type, AtAlg3-2 and Cgl-1 mutant plants.
Figure 6. Protein and N-glycosylation. Leaf protein analysis of wild type, AtAlg3-2 homozygous line and Cgl-1 mutant by Silver Staining (A) and SDS-PAGE (B). Immunoblotting using polyclonal anti-HRP antibodies that specifically recognizes complex N-glycans.
Figure 7: Western blot analysis of PDI after treatment with PNGase F
Figure 8: Western blot analysis of PDI after treatment with EndoH.

### Detailed description of the invention

The term "nucleic acid" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e. g., peptide nucleic acids). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including among other things, simple and complex cells.

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulfation, gamma-carboxylation of glutamic acid residues, hydroxylation and ADP-ribosylation.

A "coding" or "encoding" sequence is the part of a gene that codes for the amino acid sequence of a protein, or for a functional RNA such as a tRNA or rRNA and specifically refers to the fact that the nucleic acid sequence comprises the information for translation into the specified protein. A nucleic acid encoding a protein may comprise non-translated sequences (e. g., introns) within translated regions of the nucleic acid, or may lack such intervening non-translated sequences (e. g., as in cDNA). The information by which a protein is encoded is specified by the use of codons. Typically, the amino acid sequence is encoded by the nucleic acid using the "universal" genetic code. However, variants of the universal code, such as are present in some plant, animal, and fungal mitochondria, the bacterium *Mycoplasma capricolum,* or the ciliate Macronucleus, may be used when the nucleic acid is expressed therein. When the nucleic acid is prepared or altered synthetically, advantage can be taken of known codon preferences of the intended host where the nucleic acid is to be expressed. For example, although nucleic acid sequences of the present invention may be expressed in both monocotyledonous and dicotyledonous plant species, sequences can be modified to account for the specific codon preferences and GC content preferences of monocotyledons or dicotyledons as these preferences have been shown to differ.

"Expression" refers to the transcription of a gene into structural RNA (rRNA, tRNA) or messenger RNA (mRNA) with subsequent translation into a protein.

"Inhibition of gene expression" as used herein refers to the absence (or observable decrease) in the level of protein and/or mRNA product from a target gene. "Specificity" or "specific inhibition" refers to the ability to inhibit the target gene without manifest effects on other genes of the cell. The consequences of inhibition can be confirmed by examination of the properties of the cell or the organism (in the specific case of the invention, the reduction of production of glycoproteins with high mannose type N-glycans) or by biochemical techniques such as RNA solution hybridisation, nuclease protection, Northern hybridisation, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS). Basically, three methods for inhibition are known at this moment and included in this application: antisense expression, sense co-suppression and RNA-inhibition. However, the invention is not limited to these methods and any other method which causes (partial) silencing of the Alg3 gene is included.

For antisense expression, a nucleotide sequence coding for an Alg3 gene, its homologue or variant, or at least a part thereof of 40 nucleotides or more, is put behind a constitutive or tissue-specific promoter in anti-sense direction. After transcription of this nucleotide sequence an mRNA is produced which is complementary to the mRNA formed through transcription of the endogenous Alg3 gene of the plant. It is well proven by now that production of such an anti-sense mRNA is capable of at least partial inhibition of the endogenous expression of the gene for which it is complementary. Furthermore, it has been proven that to achieve this effect even sequences with a less than 100% homology are useful. Also antisense mRNA's which are shorter than the endogenous mRNA which they should inhibit can be used. Generally, it is accepted that mRNA sequences of 40 nucleotides or more which have a homology of 70% or more will be capable of generating an inhibitory effect. The principal patent reference is EP 240,208 of Calgene Inc. There is no reason to doubt the operability of antisense technology. It is well-established, used routinely in laboratories around the world and products in which it is used are on the market.

The second approach is commonly called sense co- suppression. This phenomenon occurs when the gene involved in the GA biosynthetic pathway or part of said gene is expressed in its sense direction. Although this kind of expression when full length genes are used most often results in overexpression of the gene, it has been found that in some cases and especially in cases when a sequence shorter than the full length sequence is used, expression of this gene or fragment causes inhibition of the endogenous gene. The principal patent reference on sense co-suppression is EP 465,572 in the name of DNA Plant Technology Inc.

Sense and antisense gene regulation is reviewed by Bird and Ray (Gen. Eng. Reviews 9: 207-221, 1991). Gene silencing can thus be obtained by inserting into the genome of a target organism an extra copy of the target gene coding sequence which may comprise either the whole or part or be a truncated sequence and may be in sense or in antisense orientation. Additionally, intron sequences which are obtainable from the genomic gene sequence may be used in the construction of suppression vectors. There have also been reports of gene silencing being achieved within organisms of both the transgene and the endogenous gene where the only sequence identity is within the promoter regions.

The third possible way to silence genes is by using the so-called RNAi technology, which covers all applications in which double-stranded RNAs are used to achieve silencing of an endogenous gene. As has been demonstrated by Fire et al. (Nature, 391: 806-811, 1998) application of a dsRNAi of which one strand is at least partly complementary to the endogenously produced mRNA whether produced intracellularly or added extracellularly is extremely capable of inhibiting translation of the mRNA into a protein. It is believed that this phenomenon works through the intermediate production of short stretches of dsRNA (with a length of 32 nucleotides). To achieve production of dsRNA a so-called "dsRNAi construct" is made harbouring both a sense and an antisense nucleotide sequence (together also called an inverted repeat) of at least 40 nucleotides of which one is complementary to the endogenous gene which needs to be silenced. The sense and antisense nucleotide sequences can be connected through a spacer nucleotide sequence of any length which allows for a fold back of the formed RNA so that a double stranded RNA is formed by the sense and antisense sequence. The spacer then serves to form the hairpin loop connecting both sense and antisense sequence. The order of the sense and antisense sequence is not important. It is also possible to combine more than one sense-antisense combination in one and the same construct. If the simple form is depicted as: prom - S - spac - AS - term, also the following constructs can be applied: prom - S1- spac - AS1 - spac - S2 - spac - AS2 - term, or prom - S2 - spac - S1 - spac - AS1- spac - AS2 - term. Variations in the built up of the construct are possible, as long as the end product of the transcription of said constructs yields one or more dsRNAs. Alternatively, the double stranded structure may be formed by two separate constructs coding for complementary RNA strands, where RNA duplex formation occurs in the cell. In short notation these constructs then look like: proml-S1-terml and prom2-AS1-term2. Prom1 and prom2 can be the same or different and at least one should both be a tissue-specific promoter in the current invention, term1 and term2 can be the same or different. Both constructs can be introduced into the cell on the same vector, but can also be introduced using two different vectors.

Nucleotide sequences capable of forming a dsRNA identical to a portion of the target gene are preferred for inhibition. Sequences with insertions, deletions and single point mutations relative to the target sequence have also been found effective for inhibition. Thus, sequences with a sequence identity of less than 100% may be used. Further, the duplex region of the RNA may be defined functionally as a (double stranded) nucleotide sequence that is capable of hybridising with a portion of the target gene transcript (e.g., under the conditions of 400 mM NaCl, 40 mM PIPES pH 6.4, 1 mM EDTA, 50°C to 65°C hybridisation for 12-16 hours; followed by washing). The length of the identical nucleotide sequences should be at least 40 nucleotides, but preferably larger: 50, 100, 200, 300 or 400 bases.

The term "sequence identity" as used herein denotes the presence of identity between two or more polynucleotides or between two or more polypeptides. Polynucleotides or polypeptides have "identical" sequences if the sequence of nucleotides resp. amino acids in their sequences is the same when aligned for maximum correspondence. Sequence comparison between two or more polynucleotides or polypeptides is generally performed by comparing portions of two sequences over a comparison window to identify and compare local regions of sequence similarity. The comparison window is generally from about 20 to 200 contiguous nucleotides or from about 7 to 70 contiguous amino acids. The "percentage of sequence identity" for polynucleotides or polypeptides, such as 50, 60, 70, 80, 90, 95, 98, 99 or 100 percent sequence identity may be determined by comparing two optimally aligned sequences over a comparison window, wherein the portion of the polynucleotide or polypetide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base or amino acid residue occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection. Algorithms and software suitable for use in aligning sequences for comparison and calculation of sequence homology or identity will be known to those skilled in the art. Significant examples of such tools are the Pearson and Lipman search based FASTA and BLAST programs, details of these may be found in Altschul et al (1997), Nucleic Acid Res. 25:3389-3402; Altschul et al (1990), J. Mol. Biol. 215: 403-10; Pearson and Lipman (1988), Proc. Natl. Acad. Sci. USA 85:2444-8; Lipman and Pearson (1985), Science 227:1435-41). Other suitable programs (e.g. based on the algorithms of Gribskov and Devereux, Sequence Aanlysis Primer, Stockton Press, 1991, and references cited therein) include the PILEUP, LINEUP, GAP, BESTFIT and FASTA programs in the GCG® Wisconsin Package® of the University of Wisconsin Genetics Computer Group, Madison, WI, USA, now offered through Accelrys Inc. Details of the above programs are available on the internet through 'http://www.ncbi.nlm.nih.gov/BLAST' or mirror sites and "http://www.accelrys.com/products/gcg_wisconsin_package". Thus such homology and identity percentages can be ascertained using publicly or commercially available software packages or by computer servers on the internet. By the term "identity" is meant that the stated percentage of the claimed amino acid sequence or nucleic acid sequence is to be found in the reference sequence in the same relative positions when the sequences are optimally aligned, notwithstanding the fact that the sequences may have deletions or additions in certain positions requiring introduction of gaps to allow alignment of the highest percentage of amino acids or bases. Preferably the sequence are aligned by using 10 or less gaps, ie. the total number of gaps introduced into the two sequences when added together is 10 or less. The length of such gaps is not of particular importance but generally will be no more than 10, and preferably no more than 5 amino acids, or 30 and preferably no more than 15 bases.

The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations". Every nucleic acid sequence herein that encodes a polypeptide also, by reference to the genetic code, describes every possible silent variation of the nucleic acid.

The term "complementary" in "complementary strand" means that the nucleic acid strand has a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.

The term "galactosylated N-linked glycan" refers to the common core of an *N*-linked oligosaccharide unit in glycoproteins that consists of a chitobiose core with at least three mannoses and at least one N-acetylglucosamine residue and that is further extended with at least one galactose residue on a N-acetylglucosamine at the nonreducing end.

The expression "conservative substitutions" as used with respect to amino acids relates to the substitution of a given amino acid by an amino acid having physicochemical characteristics in the same class. Thus where an amino acid in the Alg3 sequence has a hydrophobic group, a conservative substitution replaces it by another amino acid also having a hydrophobic group; other such classes are those where the characteristic group is hydrophilic, cationic, anionic or contains a thiol or thioether. Such substitutions are well known to those of ordinary skill in the art, i.e. see US 5,380,712. Conservative amino acid substitutions may be made, for example within the group of aliphatic non-polar amino acids (Gly, Ala, Pro, Ile, Leu, Val), the group of polar uncharged amino acids (Cys, Ser, Thr, Met, Asn, Gln), the group of polar charged amino acids (Asp, Glu, Lys, Arg) or the group of aromatic amino acids (His, Phe, Tyr, Trp).

The term "selection marker " refers to a polynucleotide sequence encoding a metabolic trait which allows for the separation of transgenic and non-transgenic organisms and mostly refers to the provision of antibiotic resistance. A selectable marker is for example the *aph*L1 encoded kanamycin resistance marker, the nptII gene, the gene coding for hygromycin resistance . Other selection markers are for instance reporter genes such as chloramphenicol acetyl transferase, β-galactosidase, luciferase and green fluorescence protein. Identification methods for the products of reporter genes include, but are not limited to, enzymatic assays and fluorimetric assays. Reporter genes and assays to detect their products are well known in the art and are described, for example in Current Protocols in Molecular Biology, eds. Ausubel et al., Greene Publishing and Wiley-Interscience: New York (1987) and periodic updates.

As used herein, the term "vector" includes reference to a nucleic acid used in transfection of a host cell and into which can be inserted a polynucleotide. Vectors are often replicons. Expression vectors permit transcription of a nucleic acid inserted therein.

As used herein, the term "operably linked" refers to a functional linkage or juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame.

By "host cell" is meant a cell which contains a vector and supports the replication and/or expression of the vector. Host cells may be prokaryotic cells such as E. coli, or eukaryotic cells such as plant, yeast, insect, amphibian, or mammalian cells. Preferably, host cells are bacterial cells or plant cells, more preferably plant cells.

As used herein, "heterologous" in reference to a nucleic acid is a nucleic acid that originates from a foreign species, or, if from the same species, is substantially modified from its native form in composition and/or genomic locus by deliberate human intervention. For example, a promoter operably linked to a heterologous structural gene is from a species different from that from which the structural gene was derived, or, if from the same species, one or both are substantially modified from their original form. A heterologous protein may originate from a foreign species or, if from the same species, is substantially modified from its original form by deliberate human intervention.

The term "regulatory sequence" or "control sequence" is defined herein to include any component which is necessary or advantageous for expression of a coding sequence. A regulatory sequence may be native or foreign to the coding sequence. Such regulatory sequences include, but are not limited to, a leader, a polyadenylation sequence, a propeptide sequence, a promoter, a signal sequence, and a transcription terminator. At a minimum, the regulatory sequences include a promoter, and transcriptional and translational stop signals. The regulatory sequences may be provided with linkers for the purpose of introducing specific restriction sites facilitating ligation of the regulatory sequences with the coding region of the nucleic acid sequence encoding a polypeptide.

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes. A "plant promoter" is a promoter capable of initiating transcription in plant cells whether or not its origin is a plant cell. Exemplary plant promoters include, but are not limited to, those that are obtained from plants, plant viruses, and bacteria which comprise genes expressed in plant cells such as *Agrobacterium* or *Rhizobium.* Examples of suitable promoters are the 35S promoter of Cauliflauwer mosaic virus and derivatives thereof, the ferredoxin promoter, the nopaline synthase (nos), mannopine synthase (mas) and octopine synthase (ocs) promoters (EP 0 122 791, EP 0 126 546, EP 0 145 338), the ubiquitin promoter (EP 0 342 926), the cassava vein mosaic virus promoter and the chrysanthemum promoter for the short subunit of Rubisco. A specific type of promoter to be used with the current invention is a "tissue-specific promoter". Such a tissue-specific promoter is able to drive expression of the gene to which it is coupled in certain specific tissues, while in other tissues expression is virtually absent. The tissue-specificity may be very local (e.g. confined to one particular organ of the pant, e.g. the anther or the stomata) or may be general, but with respect to a certain cell type (e.g. all epidermis cells, all phloem cells). Many of these tissue-specific promoters are known in the art, such as root specific promoters (see e.g., EP 1248850), fruit specific promoters (see e.g. US 4,943,674, US 5,536,653, US 5,753,475, EP 316441, UXS 6,566,586, EP 973922 and Schaart et al., Plant Cell rep. 2002, 21:313-319), seed-specific promoters such as the napin promoter (Stalberg et al., 1993; US 5,420,034), the endosperm-specific promoter GluB-1 (Takaiwa *et al.,* 1996), the FAE1 promoter, for example the FAE1 promoter from *Arabidopsis* (Rossak *et al.,* 2001), the oleosin promoter, for example the oleosin promoter from *Arabidopsis* (Plant *et al.,* 1994), the β-phaseolin promoter, for example the β-phaseolin obtainable from french bean (Bustos *et al.,* 1991), the glutenin promoter, such as the one obtainable from rice (Washida et al, 1999), the promoter of the *Arabidopsis thaliana* 2S albumin gene (Vandekerckhove *et al.,* 1989) and the cruA promoter of *Brassica napus* (Ryan *et al.,* 1989) and the promoters mentioned in US 5,608,152, US 5,952,489 and US 6,642,437. Further, in order to achieve specific expression in potato tubers, the patatin (B33) promoter (Rocha-Sosa et al., 1989, EMBO J., 8:23; US 5,723,757,US 5,436,393), the MOT (malate oxogluterate translocator) promoter (WO 99/06578), the promoters mentioned in US 5,436,393, US 6,184,443 and US 6,610,840, or any other promoter able to drive expression in tissues prone to sprouting such as the tuber phloem restricted version of the RolC promoter, may be used.

The term "transgenic plant or plant cell" includes reference to a plant or plant cell which comprises within its genome a heterologous polynucleotide. Generally, the heterologous polynucleotide is stably integrated within the genome such that the polynucleotide is passed on to successive generations. The heterologous polynucleotide may be integrated into the genome alone or as part of a recombinant expression cassette. Also, it is possible that the heterologous polynucleotide is not or not stably integrated in the genome of the transformed plant. In that case, the gene can be 'transiently' expressed, implying that expression occurs for a given time, after which the introduced polynucleotiode is lost from the cell. For the purposes of this invention, a transgenic plant or plant cell also includes plants or plant cells which transiently express the heterologous polypeptide. "Transgenic" is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

The term "insertion" in the context of introducing a nucleic acid into a cell, means "transfection" or "transformation" or "transduction" and includes reference to the incorporation of a nucleic acid into a eukaryotic or prokaryotic cell where the nucleic acid may be incorporated into the genome of the cell (e. g., chromosome, plasmid, plastid or mitochondrial DNA), converted into an autonomous replicon, or transiently expressed (e. g., transfected mRNA).

As used herein, the term "plant" includes reference to whole plants, plant organs (e. g., leaves, stems, roots, etc.), seeds and plant cells and progeny of same. Plant cell, as used herein includes, without limitation, seeds, suspension cultures, embryos, meristematic regions, callus tissue, leaves, roots, shoots, gametophytes, sporophytes, pollen, and microspores. The class of plants which can be used in the methods of the invention is generally as broad as the class of higher plants amenable to transformation techniques, including both monocotyledonous and dicotyledonous plants.

As described above, it is especially the "plant" character of the glycans that makes glycoproteins produced in plants less suited for pharmaceutical use. This "plant" character imparts undesirable antigenic and immunogenic characteristics to the glycoprotein in question, which would require a strategy intended to prevent immunogenicity of glycoproteins produced by transgenic plants. The aim of the strategy is to modify the genome of plant cells in such a manner that they synthesize their proteins like human cells would.

The invention was developed based on the identification of a homologue of the *S. cerevisiae* ALG3 gene of *A. thaliana* and the analysis of two different AtAlg3 mutant plants, AtAlg3-1 (Salk_064006) and ATAlg3-2 (Salk_040296c). The *Arabidopsis* Alg3 mannosyltransferase was identified and the c-DNA obtained.

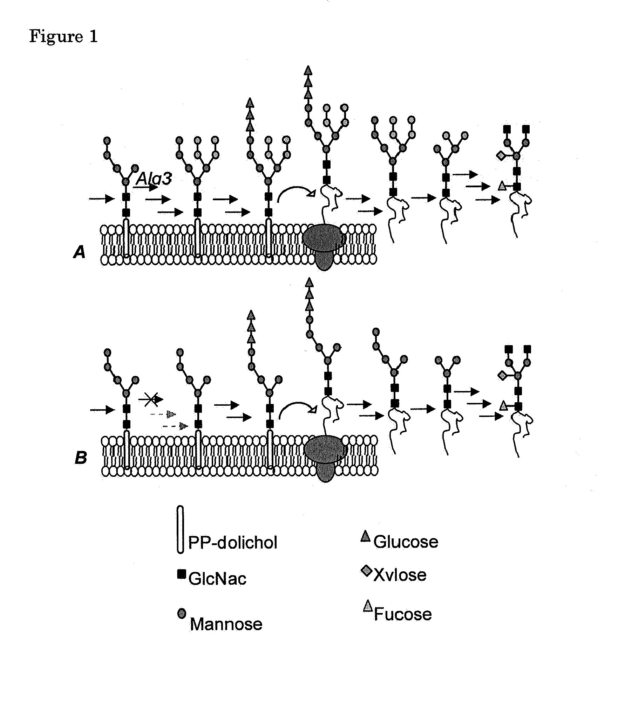

Glycan analysis of the AtAlg3-2 insertion mutant shows that more than 35% of the glycans on glycoproteins were of the Man₃GlcNAc₂ and alpha1,2 mannosidase sensitive Man₄₋₅GlcNAc₂ type in both old and young leaves (Table 1). This demonstrates the block in extension of part of the dolichol linked glycan pool to Man₅GlcNAc₂-PP-Dol and the successful transfer of these truncated glycans from dolichol to proteins (Figure 1B, alg3 pathway). No differences in the complex glycan profile between AtAlg3-2 and wildtype plants were observed and how many of the complex type glycans in the mutant originate from the Alg3 pathway remains the question. It is also currently not known however whether the intermediate Man₅GlcNAc₂-PP-Dol glycans are being glucosylated in AtAlg3-2 plants before being transferred to newly synthesized proteins. However, both in *P. pastoris* and *S*. *cerevisiae* alg3 mutant strains mono-, di- and tri- glucosylated Man₅GlcNAc₂-PP-Dol structures were detected which were also being transferred to proteins (Davidson et al, 2004; Verostek et al; 1993). Also glucosylated Man₅GlcNAc₂ oligosaccharides were isolated from different CHO cell lines (Foulquier et al, 2002; Ermonval et al 1997).
*S. cervisiae* Δalg3 strain shows a profound underglycosylation of secretory glycoproteins (Aebi et al, 1996; Huffaker and Robbins, 1983). Human patients which have a mutation in Alg3 gene show an incomplete utilization of N-glycosylation sites in glycoproteins (Körner et al, 1999). A defect in the human *ALG3* gene causes congenital disorder of glycosylation type Id (CDG-Id). At present only four patients are described in literature (Stibler et al, 1995; Denecke et al, 2004; Schollen et al, 2005; Sun et al, 2005). All known patients are homozygous or compound heterozygous for the mutation in the *Alg3* gene but have leaky expression. Fibroblasts from human Alg3 patients show an accumulation of Man₅GlcNAc₂-PP-dolichol and due to its leaky nature, a residual formation of full length lipid linked oligosaccharide (Körner et al., 1999). Surprisingly, no underglycosylation was observed on PDI and proteins containing complex glycans in AtAlg3-2 plants. Analysis of the ER resident glycoprotein PDI showed that both glycosylation sites were occupied with aberrant Man₃₋₅GlcNAc₂ glycans. This also demonstrates that all the glycans on PDI originate from the mutant pathway.

This now would mean that plants in which the expression of Alg3 is inhibited can be used for production of glycoproteins wherein the presence of high mannose type N-glycans is reduced. However, it appeared that a complete knockout of AtAlg3 is pollen lethal as is demonstrated in the mutant line AtAlg3-1. Siliques of heterozygous AtAlg3-1 mutants did not have any aborted seeds and pollen from heterozygous AtAlg3-1 plants were all shown to be viable by FDA staining. However, differences were found in pollen tube length formation between wild type and heterozygous mutant plants suggesting that mutant pollen are formed. Besides this, also the ability of mutant alg3 pollen to fertilize the ovule is prevented by the mutation. These results imply that proteins that are involved in pollen tube formation but also ovule fertilization are non-functional because of the presence of aberrant glycans. These observations are consistent with transcript profiling data in which AtAlg3 has the high expressions in mature pollen during plant development (AtGenExpress Visualization Tool, http://bbc.botany.utoronto.can. Only minimal expression of AtAlg3 is necessary to rescue the plants and to allows them to grow normally as observed in the *AtAlg3-2* insertion mutant. In *S*. *cerevisiae* and *P. pastoris* alg3 mutant strains however, complete inactivation of ALG3 genes is not lethal (Aebi et al, 1996; Davidson et al, 2004).

In contrast to N-glycan glycosylation mutants described earlier (see introduction) the homozygous AtAlg3-2 T-DNA insertion line described in this study was viable and surprisingly did not show any obvious growth phenotype under standard growth conditions. It has been demonstrated in the experimental part that this mutant is characterised as a splice mutant, having an insertion between intron 11 and exon 12. Due to this insertion the majority of the expressed proteins is expressed as a mutant protein which has no biological function in glycosylation. However, still a small amount of correctly spliced proteins are expressed and remain functional.

Therefore, the present invention shows that a partial inhibition of Alg3 is needed to achieve the desired glycosylation pattern, while being able to produce viable plants and plant cells. Such a partial inhibition can be achieved in different ways. One way would be to insert the mutant Alg3 gene derived from the AtAlg3-2 mutant into a plant cell or plant. However, the plant to be transformed also contains an orthologue of the Alg3 gene. As is demonstrated in the heterozygous mutant AtAlg3-1 the activity of the remaining wild-type Alg3 gene is sufficient to at least partly abolish the inhibitory effect of the mutant gene such that still a considerable amount of undesired high mannose type N-glycans are formed. Thus, the wild-type gene should be removed or made inoperable. This can be solved in different ways. One elegant way to achieve a desired transformed plant is by site-directed integration, whereby the mutant gene replaces the wild-type gene. A method for this site-directed integration in plants is e.g. described in US 5,501,967. Such a site-directed integration can be performed with a construct harbouring the complete mutant alg3 gene, but also the part comprising the location of the mutation, thus the region comprising intron 11 and exon 12, can be used for this purpose. Alternatively, a splice mutant can be generated by site-directed integration of a heterologous stretch of DNA between intron 11 and exon 12. preferably said inserted heterologous stretch of DNA contains T-DNA borders, which appear to interfere with the normal splicing mechanism in plants. In this way a heterozygous plant is made, which, however, will still give rise to undesired high mannose type N-glycans. To solve this, the transformed plant is further cultivated and plants homozygous for the mutant gene are obtained by self-crossing the heterozygous plants. Alternatively, the wild-type genes can be removed or made inoperable by a knock-out event. Of course, in this case care should be taken that the mutant Alg3 gene is not hampered by such a knock-out event and still is expressed properly. Alternatively, expression of the endogeneous Alg3 enzyme can first be inactivated after which a mutant alg3 gene is introduced into the plant.

In another embodiment of the present invention partial inhibition of the alg3 gene is accomplished by site-specific silencing. As has been explained above, basically three methods are available to achieve silencing in plants, i.e. the anti-sense inhibition, the sense co-suppression and double-stranded RNA inhibition (RNAi or dsRNAi). For the first two methods, anti-sense inhibition and sense co-suppression, it is known that quite often only partial inhibition is obtained, which is desirable for the present invention. As is known to the person skilled in the art, and as is extensively described in the plant genetic engineering art, a construct should be made harboring part or all of the gene to be silenced, in this case the endogenous Alg3 gene.

First of all, it should be submitted that a person skilled in the art is able to find the endogenous (orthologous) Alg3 gene in a plant or plant cell of interest on basis of the sequence data given herein. The specific primers as mentioned in the experimental part can be used to locate, PCR and clone such a gene. Possibly, the primers need to be adapted to allow for a more liberal specificity by introducing inositine moieties in the primer sequences. Such an approach is known and well within the skill of a researcher in this field. Once the endogenous gene of a specific plant or plant species is identified, the sequence is determined and used to construct an anti-sense or sense construct. As indicated above, for both types of constructs, the complete coding sequence or a fragment of the coding sequence can be used. The fragment should be large enough to have the intended silencing effect, i.e. the sense or anti-sense sequence should preferably have a length of 50 nucleotides or more, more preferably 100 nucleotides or more, more preferably 200 nucleotides or more and most preferably a length of 300 nucleotides or more. A constitutive promoter can be used to drive expression of the anti-sense or sense gene or fragment thereof. It is, however, also possible to operably link the sense or anti-sense coding sequence to a tissue specific promoter (discussed in more detail below). Both promoter and sense or anti-sense sequence are operably connected and inserted into a cloning vector for the transformation of the plant.

In the case of RNAi inhibition in general will be complete, and thus to achieve partial inhibition a tissue-specific promoter is needed. Since it appears that the lethal effects of the complete Alg3 knock-out are accomplished by defective pollen and/or defective pollen tube formation, such a tissue-specific promoter preferably should not be inducing expression in pollen tissue. Further, for economic reasons of transgenic protein production in plants, the inhibition should be effected in plant tissues which are easily harvestable and which can comprise large amounts of the produced glycoproteins. Thus, preferably, the tissue-specific promoter should be selected from promoters able to drive expression in storage and sink organs such as tubers, seeds and leaves.

The dsRNAi construct should, similar to the sense and anti-sense constructs, be based on the endogenous Alg3 sequence of the plant in which the Alg3 expression needs to be silenced. As is generally known in the field of RNAi, such a construct should be able to express dsRNA which is highly homologous or identical to the target gene.

Thus also included in the invention are constructs having a nucleotide sequence under control of a tissue-specific promoter wherein said nucleotide sequence comprises a part of 40 or more nucleotides in a sense direction, or in an antisense direction or in an inverted repeat form, of the sequence of the Alg3 gene, or orthologues or homologues thereof.

The recombinant DNA constructs for use in the methods according to the present invention may be constructed using recombinant DNA technology well known to persons skilled in the art. The recombinant gene constructs may be inserted into vectors, which may be commercially available, suitable for transforming into plants and suitable for expression of the gene product in the transformed cells. Preferably used are binary vectors which are useful for plant transformation using *Agrobacterium.*

Selectable markers, which may be included as a part of the introduced recombinant DNA, are used to select transformed cells (those containing recombinant DNA) over untransformed cells. Examples of suitable markers include genes that provide antibiotic or herbicide resistance. Cells containing the recombinant DNA are capable of surviving in the presence of antibiotic or herbicide concentrations that kill untransformed cells. Examples of selectable marker genes include the bar gene which provides resistance to the herbicide Basta; the nptII gene which confers kanamycin resistance; the hpt gene which confers hygromycin resistance; and the cah gene which gives resistance to cyanamid. An entire plant can be generated from a single transformed plant cell through cell culturing techniques known to those skilled in the art. Also transformation techniques for obtaining marker-less plants (such as described in WO 02/097102) may be employed.

Next, the transgenic plants of the present invention may additionally comprise a heterologous nucleotide sequence coding for a glycoprotein of interest of which it is desired to reduce the amount of said glycoproteins which are carrying high mannose type glycans according to the present invention. Such a protein is preferably a human protein such as a human or humanised antibody. The nucleotide sequence for this protein can be introduced together with the nucleotide sequence for the Alg3 mutant gene and/or the sequence for silencing the endogenous Alg3 gene, both on the same vector or on two different vectors, or it may have been provided before or after the plant was transformed for altered glycosylation. Alternatively, plants with reduced Alg3 expression and reduced in high mannose type N-glycans can be used as hosts to produce heterologous glycoproteins via transient expression using for example but not limited to viral vectors as described by Giritch et.al. Proc Natl Acad Sci U S A 103:14645-6, 2006.

The invention to reduce the amount of high mannose type N-glycans on glycoproteins might also be particularly useful when said glycoproteins are expressed or targeted to tissues and/or subcellular compartments that would normally result in high mannose type glycans on said glycoproteins. Examples include but are not limited to targeting of said glycoproteins by ER retention signals such as KDEL (Petrucceli *et.al,.* 2006) and targeting of said glycoproteins by peptides derived from maize endosperm protein storage proteins (zeins) in structures that originate from the endoplasmic reticulum (ER) and contain typical ER high mannose type glycan profiles.

In the following Examples the invention is illustrated in more detail. The embodiments of the inventions are, however, in no way limiting the scope of the invention.

### Examples

### Example 1 Reducing high mannose glycoproteins in Arabidopsis

### Plant materials and growth conditions

Seeds of *Arabidopsis thaliana* Columbia-0 *(Arabidopsis)* and T-DNA insertion lines were sown on 9 cm 0.8% daishun agar Petri dishes and placed in a cold room at 4°C for 2 days in the dark to promote uniform germination. Germination and plant culture were performed in a climate chamber (20°C/15°C day/night temperatures; 250 µmol light m⁻² s⁻¹ at plant level during 12 h/d and 75% relative humidity).

### Isolation of Genomic DNA

Plant material was collected in eppendorf tubes and ground with a pestle in liquid nitrogen and 400µl DNA isolation buffer (5 M Urea, 0.3 M NaCl, 50 mM Tris pH 7.5, 20 mM EDTA, 2% N-Lauroyl sarcosine, 0.5% Sodium Dodecyl Sulfate (SDS), 5 % phenol pH 8.0 (0.1% 8-hydroxyquinolin)) and 400 µl Phenol:chloroform:isoamylalcohol solution (1:1:0.02). The supernatant containing the DNA was precipitated with isopropanol and washed twice in 70% ethanol. The DNA was dissolved in 50 µL TE (10 mM Tris-HCl and 1 mM EDTA, pH 8.0) containing 10 µg/ml RNase A.

### Identification and genotyping of T-DNA insertion lines

*Arabidopsis thaliana* T-DNA lines Salk_064006 and Salk_040296c from the SALK institute were obtained via the Nottingham Arabidopsis Stock Centre (NASC). AtAlg3-1 (from Salk_064006) and AtAlg3-2 (from Salk_040296c) were screened by PCR on genomic DNA using the forward primers Alg3-F2.2 (5'-CGTTCAGTGATGTATCAGCCTCACGG-3') and Alg3-F3 (5'-AAGCGTTGTCCATTTACGATG-3') and the reverse primers Alg3-R2 (5'-GGATTTAGGGTGTTCTTTGAGCTGATAAGGr-3') and Alg3-R3 (5'-CTTATAATTTGCGAACGCCAC-3') as well as the T-DNA specific LBa1 or LBb1 primers. PCR products were sequenced to determine the exact insertion site.

### SDS-PAGE and immunoblotting

Plant material was ground in liquid nitrogen, resuspended in 10 µl phosphate-buffered saline buffer (137 mM NaCl, 2.7 mM KCl, 10 mM Na₂HPO₄, 2 mM KH₂PO₄, pH 7.4) per mg of plant material and cleared by centrifugation. An aliquot of the supernatant was immediately mixed with SDS-polyacrylamide gel electrophoresis (PAGE) loading buffer, denatured at 95°C for 5 min and subjected to 8 or 12.5% SDS-PAGE under reducing conditions. Western blotting was performed with PVDF membranes, and blocked with 5% (w/v) non-fat dry milk in Tris-buffered saline (TBS, 20 mM Tris^HCl; pH 7.6, 137 mM NaCl). The membranes were probed either with anti-HRP (1:5000; Sigma) of anti-PDI (1:3000; Rosebiotech). Detection of bound primary antibodies was performed with either BCIP/NBT or Lumi-Light and Lumi-Imager (Roche Diagnostics) after incubation with goat anti-rabbit antibodies.

### Deglycosylation with PNGase F and endoglycosidase H

PNGase F and Endo H were purchased from New England Biolabs (NEB) and used according to the included methods using NEB's recommended buffers. The reactions were terminated by boiling in SDS-polyacrylamide gel electrophoresis (PAGE) loading buffer.

### RNA isolation and RT-PCR analysis

RNA was extracted from *AtAlg3-2,* wildtype and *Cgl-1* mutant leaves using TriPure Isolation Reagent (Roche) and concentration was measured with NanoDrop® ND-1000 UV-Vis Spectrophotometer. RNA was used to make cDNA with Taqman Reverse Transcript reagent (Applied Biosystems). Quantitative real-time PCR was performed using the Bio-Rad iQSYBR Green Supermix single color detection system. Reactions were set up in triplicate. Analysis was performed using the supplied software program using *actin 2* as a reference control. Primers for quantitative PCR were designed and produced single amplification products as determined by melt-curve analysis. The following pair of primers were used: for actin, 5'-GGTAACATTGTGCTCAGTGGTGG-3' and 5'-AACGACCTTAATCTTCATGCTGC-3'; for AtUTR2, 5'-CACATTTATCGGTCAAGTCTCCGTT-3' and 5'-TCGCAGGAG GCGATGGTGATAGAG-3'; for upstream of the T-DNA insertion, 5'-CGCACTTATTATCGCCTATGTTC-3' and 5'-GCCCTGTATCGCCTTTCAAG-3'; for downstream of the T-DNA insertion, 5'-ACTTCTATTCGCTACCTTATCTAC-3' and 5'-GGAGGACGGTGTTGATGG-3'. For PCR analysis of Alg3-2 and wildtype splicing of intron 12 the Alg3-F2.2 and Alg3-R2 primers were used.

### Isolation of N-glycans

Proteins for N-glycan purification was extracted from 250 mg of leaf material and bound N-glycans were released by PNGase A (Roche) as described previously (Bakker et al., 2006*).

### Mannosidase Digestion of Glycans

Aspergillus satoi α1,2-mannosidase (10mU/ml; Pro-enzyme, Glyko) digestions were performed in the supplied buffer for 24h at 37°C. Reactions were then diluted with 0.5 ml of milliQ water and desalted by passage through a 500-mg C18 column.

### N-Glycan Analysis

Purified N-glycans were dissolved in 5mM NaAc and mixed with an equal volume of 1% 2,5-dihydroxybenzoic acid in 50% acetonitrile. One-microliter aliquots were spotted onto a stainless-steel sample plate and dried under a stream of air at room temperature. Positive-ion MALDI-TOF spectra of [M+Na]⁺ adducts were recorded on an Ultraflex mass spectrometer (Bruker, Billerica, MA) fitted with delayed extraction and a nitrogen laser (337 nm). A maltodextrin series was used as an external molecular weight standard. Spectra were generated from the sum of 200-300 laser pulses.

### Results

### Identification of Arabidopsis ALG3 gene

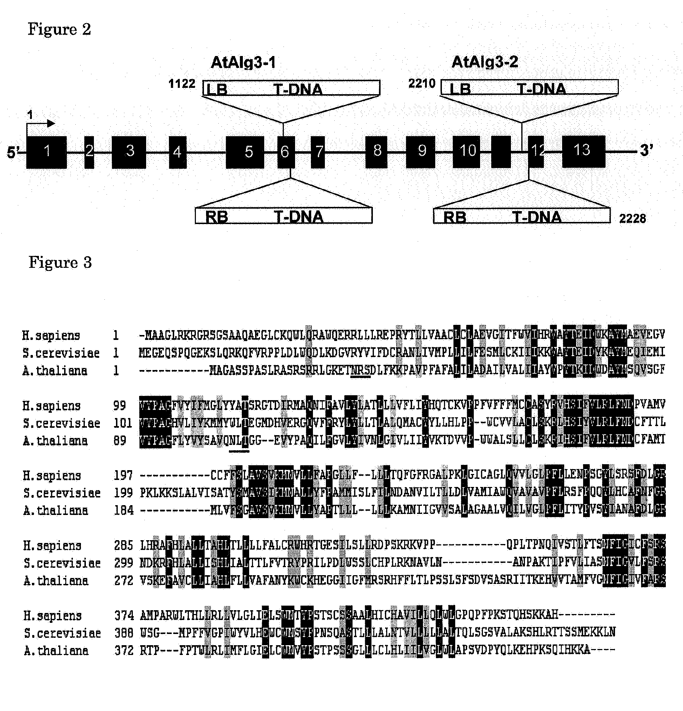

The ALG3 gene from *Saccharomyces cerevisiae* and the human Not (neighbour of tid) 56-like gene encode a mannosyltransferase involved in the first glycan elongating step in the ER, resulting in the synthesis of the Man₆GlcNAc₂-PP-Dol (Aebi, M. et al., 1996, Glycobiology 6:439-444; Korner, C. et al., 1999, EMBO J. 18:6816-6822). Inspection of the *Arabidopsis* protein and genomic databases, based on amino acid sequences of the human Not56L and *S. cervisiae* Alg3 proteins, resulted in the identification of a putative orthologue encoded by the *Arabidopsis thaliana* genome (At2G47760). The c-DNA encoding the putative Alg3 orthologue was obtained from Arabidopsis Biological Resource Centre (ABRC, clone U14893) and its sequence was confirmed and designated here as *AtAlg3.* Comparison of the cDNA with the genomic sequence shows that the gene encodes a pre-messenger with 12 introns and that the mRNA encodes a protein of 439 amino acid residues (Fig 2). Sequence homology to the human Not56 and *S.cerevisiae* Alg3 protein is relatively low (40% and 29% sequence identity respectively, Figure 3) and reveals 2 potential glycosylation sites in the AtAlg3 protein which are not conserved. Analysis of the AtAlg3 protein using TMHMM Server (v2.0) predicts 10 possible transmembrane helices. The AtAlg3 protein contains a C-terminal KKA sequence, similar to the KKXX (X = any amino acid) which were shown to be involved in retrieving transmembrane proteins from the Golgi to the endoplasmic reticulum in mammalian cells as well as in yeast (Benghezal et al, **2000***).

### Isolation and characterization of a T-DNA-tagged AtALG3 mutant

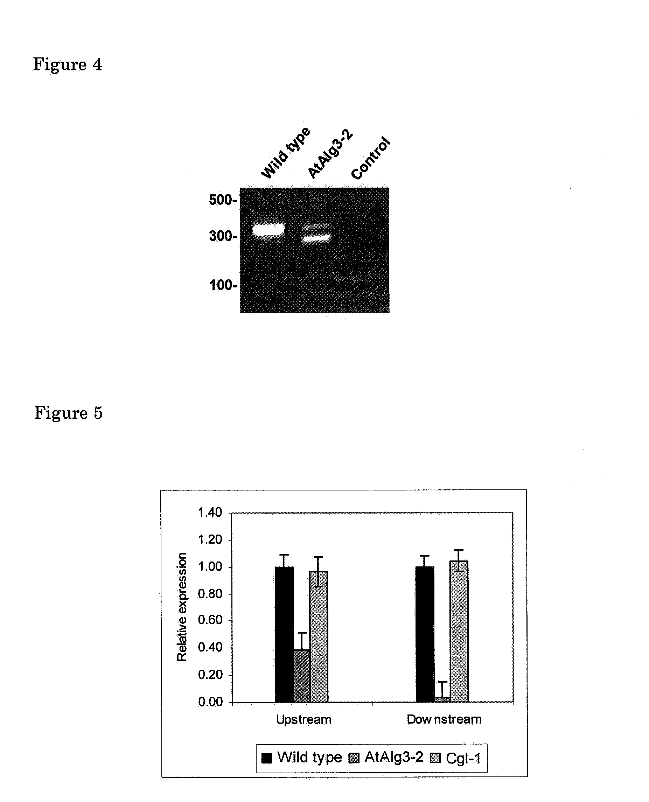

In order to study the role of the AtAlg3 gene we screened the sequence-indexed *Arabidopsis* T-DNA insertion lines from the Salk institute collection for T-DNA insertions in the AtAlg3 gene. We analyzed two lines (Salk_064006 and Salk_040296c) with T-DNA insertions in different locations in the At2g47760 locus, of which the Salk_040296c line was available as homozygous line. The T-DNA insertion in the AtAlg3 gene, designated here as *AtAlg3-1* in the Salk-064006 line was mapped in codon 199 in the 6th exon by sequence analysis of PCR fragments amplified by using T-DNA and the AtAlg3 specific forward primer. This insertion at the 5' end of the coding sequence strongly suggests that this gene is inactivated by the insertion. We subsequently tried to obtain plants homozygous for the insertion. When sawn on non-selecting media, all of 80 seeds from a heterozygous plant germinated. However, of these 80 plants tested by PCR analysis, 36% heterozygous and 64% wild type and no plants homozygous for this insertion were obtained suggesting that knock-out of both alleles is lethal. The observation that all seeds had germinated and that no aberrant seeds were found in the seedpods of these off-spring plants, suggest that not ovule development but the formation of fertile pollen is prevented by the mutation.
In contrast, homozygous plants were obtained from the Salk-040296c line. The insertion of T-DNA in the *AtAlg3* gene, designated here as *AtAlg3-2,* of this line was analysed and the results showed that this gene carries at least has two T-DNA insertions in intron11, one of which precisely between intron 11 and exon 12 (figure 2). Lack of splicing of intron11 would result into a transcript with a stop codon immediately downstream of exon11 encoding a c-terminally truncated 361 amino acid residues protein that lacks 2 putative membrane spanning segments. As could be expected, such transcripts appear not to encode a functional enzyme since a c-DNA with a stop codon engineered immediately downstream exon11 does not complement the yeast deletion mutant (data not shown). Surprisingly, this AtAlg3-2 mutant did not show any obvious growth phenotype under standard conditions. The data suggest rescue of functional AtAlg3 mRNA by splicing of intron 11 containing the T-DNAs. To confirm the presence of correctly spliced mRNA we preformed a PCR analysis on cDNA from wild type and mutant leaves using primers flanking the putative splice site and hybridize on exon11 and exon13. For the mutant leaves, this resulted in two bands on gel (Fig. 4, lane 2) of which the upper band co-migrated with the fragment amplified from wild type leaves (Fig. 4, lane 1). Both bands amplified from the mutant leaves were isolated and sequence analysis of the upper band demonstrates that indeed correct splicing of intron11 containing the T-DNA inserts occurs. The lower PCR band appeared to be the result of an aberrant splicing which couples exon11 in frame to exon13. This aberrant in frame splicing, also known as exon skipping, is apparently triggered by the T-DNA insertion since it is not observed in wild type plants (figure 4, lane 1).
To quantify the amount of correctly spliced AtAlg3 mRNA, we performed real time PCR experiments with two primer pairs hybridizing either upstream or immediately downstream of the T-DNA insertion on the c-DNA from mutant plants and control plants. The results with the upstream primer pair showed that, as compared to wild type plants about, 40% of AtAlg3 specific mRNA variants are present in the homozygous mutant. However, the experiments with the primer pair hybridizing immediately downstream of intron 11 demonstrate that this intron is not spliced from the majority of these mRNA variants and that correctly spliced mRNA accumulates in AtAlg3-2 plants to 4% of that in wild type plants. As expected, AtAlg3 mRNA levels are not affected in *cgl-*1 plants which are unable to synthesize complex glycans due to a mutation far more downstream of AtAlg3 in the N-linked glycosylation pathway. (Figure 5)

### Protein glycosylation in the Arabidopsis Alg3 mutant.

In order to study the effect of the strongly reduced AtAlg3 expression on N-glycan biosynthesis, N-glycans were released by PNGaseA treatment from glycoproteins isolated from young and old leaves of homozygous *AtAlg3-2* plants and wild-type plants, and subsequently analyzed by Maldi-TOF (table 1). In general, there appears to be very little difference between the N-glycans isolated from old and young leaves. Results show that the complex glycan profile of the mutant is very similar to that of wild type plants. However, no high mannose type glycans were detected and, instead of accumulating Man₅GlcNAc₂ glycans, the mutant plant accumulated Man₃GlcNAc₂ and Man₄GlcNAc₂ glycans. This is in line with the hypothesis that an aberrant Man₅GlcNAc₂ structure accumulates, which is partially converted via Man₄GlcNAc₂ to Man₃GlcNAc₂ by alpha, 1-2 mannosidases present in the Golgi (figure 1B). The remaining amount of Man₅GlcNAc₂ glycans found in mutant plants may be of the isoform having alpha1-2 mannose residues (figure 1B) or of the isoform characteristic for wild type plants which does not posses alpha1-2 linked mannose residues (figure 1A). To distinguish these isoforms, the N-glycan samples were incubated with a1-2 mannosidase and analysed by MALDI-TOF. As expected, all high mannose type glycans having six or more mannose residues disappear from the sample, demonstrating the effectiveness of the alphal-2 mannosidase treatment. Also Man₄GlcNAc₂ disappears, proving it to be the isoform indicative for an abnormal biosynthesis route such as depicted in figure 1b in which extension of the 1-6 arm on the glycolipid is prevented by lack of AtAlg3 mannosyltransferase activity. The observation that despite the efficient in vitro trimming by alphal-2 mannosidase of high mannose and Man₄GrlcNAc₂, at least part of the Man₅GlcNAc₂ glycans from mutant plants are alphal-2mannosidase resistant, demonstrates residual AtAlg3 mannosyltranferase activity and is in line with the leaky AtAlg3 mRNA expression of the *AtAlg3-2* mutant.

The accumulation of Man3GlcNAc2 and particularly of the alpha1,2 mannosidase sensitive isoform of Man4GlcNAc2 on glycoproteins in the homozygous *AtAlg3-2* mutant demonstrates the block in extension of part of the dolichol linked glycan pool and the successful transfer of these truncated glycans from dolichol to proteins. In yeast the alg3 mutation and accumulation of truncated dolichol glycans result in underglycosylation of glycoproteins. We therefore investigated whether the efficiency of transfer of truncated dolichol linked glycans is affected in plant cells as well. Proteins from the *AtAlg3-2* mutant, from wild-type and from the complex-glycan less mutant *cgl*-1 were isolated from leaves and separated by SDS-PAGE. The *cgl-*1 mutant plants

**Table 1: Relative amounts of N-glycans detected in Arabidopsis thaliana wild type and Alg3 mutant. Percentages were calculated based on peak areas from matrix-assisted laser-desorption ionization-time-of-flight (MALDI-TOF) mass spectra.**

| | *Col young* | *Col old* | *Col old manI* | | *Alg3 young* | *Alg3 old* | *Alg3 old ManI* |
|---|---|---|---|---|---|---|---|
| m/z | | | | | | | |
| *(M + Hybrid-type and complex-* | *% of* | *% of* | *% of* | | *% of* | *% of* | *% of* |
| *Na)+ type structures* | *total* | *total* | *total* | | *total* | *total* | *total* |
| *1065.4 Man₃XylGlcNAc₂* | *8.3* | *5.0* | 1.7 | | *6.8* | *7.3* | *4.7* |
| *1211.4 Man₃XylFucGlcNAC₂* | *15.3* | *11.3* | *13.1* | | *16.1* | *19.5* | *21.7* |
| *1268.5 GlcNAcMan₃XylGlcNAc₂* | *8.5* | *7.7* | *3.5* | | *5.8* | *6.8* | *4.6* |
| *1414.5 GlcNAcMan₃XylFucGlcNAc₂* | *8.7* | *9.1* | *12.5* | | *10.6* | *11.9* | *17.1* |
| *1617.6 GlcNAc₂Man₃XylFucClcNAc₂* | *15.1* | *15.0* | *23.7* | | *18.7* | *16.7* | *27.3* |
| *(Oligo)mannosidic structures* | | | | | | | |
| *933.3 ManaGlcNAc₂* | | | | | *24.6* | *21.1* | *22.1* |
| *1095.4 Man₄GlcNAc₂* | | | | | *11.8* | *11.4* | |
| *1257.4 Man₅GlcNAc₂* | *26.7* | *31.7* | *45.5* | | *5.6* | *5.4* | *2.6* |
| *1419.5 Man₆GlcNAc₂* | *4.7* | *7.5* | | | | | |
| *1581.6 Man₇GlcNAc₂* | *4.5* | *5.7* | | | | | |
| *1743.6 Man₈GlcNAC₂* | *5.9* | *5.2* | | | | | |
| *1905.7 Man₉GlcNAc₂* | *2.2* | *1.8* | | | | | |

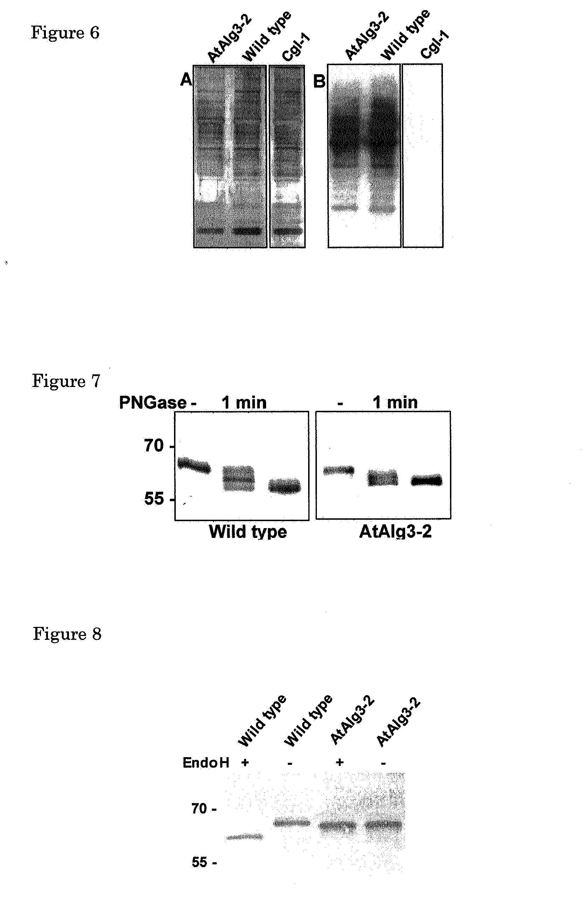

lack N-acetylglucosaminyltransferase I (GNT-I) activity and consequently do not posses complex N-glycans. The presence of proteins with plant complex glycans that carry xylose and/or 1,3 fucose can be detected on the Western blot with anti-HRP antibodies. The results show that the profile of proteins with N-linked complex glycans is very similar in the homozygous AtALG3 mutant strain and WT plants. As expected no complex N-glycans were detected in *cgl*-1 plants (figure 6).
Since glycosylation in the mutant differs mainly with respect to high mannose N-glycans, we investigated the glycosylation of the ER resident, high mannose glycoprotein PDI (protein disulfide isomerase), by studying its electrophoretic mobility via SDS-PAGE and western blotting. Both potential glycosylation sites on PDI are glycosylated in wild type plants, as well as in the mutant AtAlg3-2 plants as is visualized by the step wise removal of the N-glycans after limited PNGase F treatment (Fig.7 , lanes 1-3 and 4-6 respectively). The N-glycans of PDI from wild type plants can also be removed by EndoH as could expected form high mannose glycans present on ER resident glycoproteins (Fig 8, lane 1). However, N-glycans on PDI in mutant plants are resistant to EndoH (Fig. 8, lane 3), showing that these are not high-mannose N-glycans but probably N-glycans of the Man₅GrlcNAc₂ to Man₃GlcNAc₂ isoforms.

### Example 2: Silencing of Nicotiana tabacum alg3 gene.

### pCASintron:

A DNA fragment comprising a *Arabidopsis thaliana* ubiquitin-10 intron (SEQ UBQ10) will be amplified using PCR with primers 5'-GTGACGAGCTCGTAAATTTCTGTGTTCCTTATTCTCTC-3' and 5'-GTGACAAGCTTCTGTTAATCAGAAAAACTCAGATTAATC-3' from A. *thaliana* genomic DNA. The resulting fragment will be digested with *Sac* I and *Hin* dIII and cloned into likewise digested pCASesp. pCASesp is a pUC19 derivative in which the *Hin* dIII and *Eco* RI sites flanking the multiple cloning site have been used to insert the sequence SEQ CASesp at the same time removing these two sites and the *Eco* 31I-site in the backbone. Thus, vector pCASintron will arise.

### pCASalginv:

A cDNA fragment comprising part of the tobacco *alg3* gene (SEQ NtALG3) will be amplified using RT-PCR with primers (5'-GTGACCATGGrATGCTTATATGTCTCAGGTTAC-3' and 5'-GTGACGAGCTCAGAAGTGGATGAAAACACGACC-3') from cDNA prepared from *N. tabacum* cv Samsun total leaf RNA. The resulting fragment will then be digested with *Nco* I and *Sac* I and cloned into plasmid pCASintron digested with *Nco* I and *Sac* I and treated with CIAP. Subsequently a clone pCASalgpart, containing a sense fragment from the tobacco *alg3* upstream of the intron, will be selected and its DNA digested with *Bam* HI *and Xba* I and then treated with CIAP. This DNA will be used to insert a tobacco *alg3* PCR fragment in the inverted position with respect to the *alg3* fragment that is already present. The required fragment will amplified using RT-PCR and cDNA prepared from *N. tabacum* cv Samsun total leaf RNA with primers 5'-GTGACGGATCCATGCTTATATGTCTCAGGTTAC-3' and 5'-GTGACTCTAGAAGTGGATGAAAACACGACC-3'. This fragment will be digested with *Bam* HI and Xba I and cloned into the vector pCASalgpart containing part of the tobacco *alg3* cDNA gene. The resulting clone pCASalginv will contain an *A*. *thaliana* intron flanked by an inverted repeat made up of part of the tobacco *alg3* gene.

### pRAPalginv:

Plant transformation vectors containing the inverted *alg3* repeat gene can be made by first cloning the gene digested with *Nco* I and *Bam* HI into vector pRAP40 digested with *Nco* I and *Bam* HI causing the genes to be downstream of the enhanced CaMV 35S promoter and the AMV translational enhancer. pRAP40 is a pUC19 derivative containing the enhanced CaMV 35S promoter and the nos terminator flanked upstream of the promoter by the *Asc* I site and downstream of the terminator by the *Pac* I site; the entire cassette including the flanking restriction sites is described under SEQ RAP40. The cassette comprising the promoter, the *alg3* inverted repeat gene and the terminator will then be transferred to a modified version of binary vector pMOG22 in which the *Eco* RI and *Hin* dIII sites of the multiple cloning site had been replaced by *Pac* I and *Asc* I, respectively (Goddijn et al., 1993, Plant J 4:863-873). To this end, the pRAP-derived clones will be digested with *Pac* I and *Asc* I and then cloned into Asc-Pac digested binary vector giving a vector ready for plant transformation. After *Agrobacterium tumefaciens-*mediated transformation of *Nicotiana tabacum* , transgenic plants expressing the inverted repeat *alg3* gene can be selected using MALDI TOF analysis of the N-glycans synthesized by the transformants using methods as described previously by Bakker et al., (Proc Natl Acad Sci U S A 98:2899-904, 2001 and Proc Natl Acad Sci U S A 103:7577-82, 2006), for example. MALDI-TOF analysis of purified N-glycans from plants expressing the inverted *alg3* gene will allow to select for plants that show the desired decrease in the level of high-mannose glycoforms, e.g. Man9 to Man5 structures.

SEQ UBQ10:
GTAAATTTCTGTGTTCCTTATTCTCTCAAAATCTTCGATTTTGTTTTCGTTCGATC
CCAATTTCGTATATGTTCTTTGGTTTAGATTCTGTTAATCTTAGATCGAAGACGAT
TTTCTGGGTTTGATCGTTAGATATCATCTTAATTCTCGATTAGGGTTTCATAGATA
TCATCCGATTTGTTCAAATAATTTGAGTTTTGTCGAATAATTACTCTTCGATTTGT
GATTTCTATCTAGATCTGGTGTTAGTTTCTAGTTTGTGCGATCGAATTTGTCGATT
AATCTGAGTTTTTCTGATTAACAG

SEQ CASesp:
GGCGCGCCTCGAGGCGATCGCAGATCTATCGATGCATGCCATGGTACCCGGAG
CTCGAATTCTAGAAGCTTCTGCAGACGCGTCGACGTCATATGGATCCGCGAGAGA
CGTCTTAATTAA

SEQ RAP40:
GGCGCGCCAAGCTTGAATTAATTCTACTCCAAAAATATCAAAGATACAGTCTCAG
AAGACCAAAGGGCAATTGAGACTTTTCAACAAAGGGTAATATCCGGAAACCTCCT
CGGATTCCATTGCCCAGCTATCTGTCACTTTATTGTGAAGATAGTGGAAAAGGAA
GGTGGCTCCTACAAATGCCATCATTGCGATAAAGGAAAGGCCATCGTTGAAGATG
CCTCTGCCGACAGTGGTCCCAAAGATGGACCCCCACCCACGAGGAGCATCGTGG
AAAAAGAAGACGTTCCAACCACGTCTTCAAAGCAAGTGGATTGATGTGATAATTC
CGCATGGAGTCAAAGATTCAAATAGAGGACCTAACAGAACTCGCCGTAAAGACT
GGCGAACAGTTCATACAGAGTCTCTTACGACTCAATGACAAGAAGAAAATCTTCG
TCAACATGGTGGAGCACGACACACTTGTCTACTCCAAAAATATCAAAGATACAGT
CTCAGAAGACCAAAGGGCAATTGAGACTTTTCAACAAAGGGTAATATCCGGAAAC
CTCCTCGGATTCCATTGCCCAGCTATCTGTCACTTTATTGTGAAGATAGTGGAAA
AGGAAGGTGGCTCCTACAAATGCCATCATTGCGATAAAGGAAAGGCCATCGTTG
AAGATGCCTCTGCCGACAGTGGTCCCAAAGATGGACCCCCACCCACGAGGAGCA
TCGTGGAAAAAGAAGACGTTCCAACCACGTCTTCAAAGCAAGTGGATTGATGTGA
TATCTCCACTGACGTAAGGGATGACGCACAATCCCACTATCCTTCGCAAGACCCT
TCCTCTATATAAGGAAGTTCATTTCATTTGGAGAGGGTTTTTATTTTTAATTTTCT
TTCAAATACTTCCACCATGGGCGAGCTCGGTACCCGGGGATCCCCGGGTACCGA
GCTCGAATTTCCCCGATCGTTCAAACATTTGGCAATAAAGTTTCTTAAGATTGAAT
CCTGTTGCCGGTCTTGCGATGATTATCATATAATTTCTGTTGAATTACGTTAAGCA
TGTAATAATTAACATGTAATGCATGACGTTATTTATGAGATGGGTTTTTATGATTA
GAGTCCCGCAATTATACATTTAATACGCGATAGAAAACAAAATATAGCGCGCAAA
CTAGGATAAATTATCGCGCGCGGTGTCATCTATGTTACTAGATCGGGAATTCCAG
ATCTGCGGCCGCTTAATTAA

SEQ NtALG3:
GGATGCTTATATGTCTCAGGTTACTGGCTTTCTTGAAGGAGAGAGGGATTATAGT
AACTTGAAAGGTGATACAGGGCCTCTAGTTTATCCAGCAGGCTTTCTTTATATTT
ACTCTGCTATTCAATACGTTACCGGAGGTCAAGTCTATCCTGCTCAGATTCTTTTT
GGCTTTCTCTACATGCTGGATCTAGCAATTGTCGTGTTCATCTACTTGAAGACTG
ATGTGGTACCTTGGTGGGCTCTTTCCTTGCTTTCTCTGTCTAAAAGAGTTCACTCT
ATCTTTGTTCTTCGATTATTTAATGATTGTTTAGCCACTACTCTCCTCCATGCTGC
ATTGATCTCAATTATTTATCAAAAATGGCATCTAGGGTTGGTAATTTTCAGTGGA
GCTGTTTCTATAAAGATGAATGTGCTCCTGTATGCACCACCTCTGTTGCTCCTGA
TGGTGAAGGCAATGGATATTGTTGGAGTTATATCTGCTTTAGCAGGTGCTGCATT

AGTGCAGATTGTCATTGGGCTGCCTTTTGTCCTGTCACATCCAGCTTCATATATAT
CAAAAGCTTTCGATCTTGGTCGTGTTTTCATCCACTTCT

### Example 3 Production of monoclonal antibody in Alg3 tobacco plant or cell culture

In a preferred embodiment a transgenic Alg3-plant (Nicotiana tabacum, Oryza sativa, other) as described herein, can be used for the production of a glycoprotein, preferably a recombinant glycoprotein such as but not limited to a monoclonal antibody. For the production of a monoclonal antibody such as MGR48 (NCBI entry AY311598 for light chain and AY311599 for heavy chain sequence) in such a plant, the resulting alg3-plant such as the tobacco alg3-plant described in Example 2 needs to be transformed with a gene construct comprising an expression cassette comprising the MGR48 light and heavy chain coding sequences with appropriate controlling elements such as promoter and terminator sequences as described (Elbers et al., 2001; Plant Physiol. 2001 (126):1314-1322) or otherwise as known by those skilled in the art. Resulting transgenic plants can be analysed for the expression of the monoclonal antibody as described and a cell suspension culture can be derived from a selected alg3 transgenic tobacco plant expressing properly folded monoclonal antibody. A cell suspension culture can be made by incubating leaf material of the selected plant on solidified MS-medium (Murashige and Skoog, 1962) supplemented with 1 mg/l 2,4-D at 25°C to obtain friable callus material. Callus material can be grown in liquid medium to obtain a cell culture which can be used for the manufacture of the monoclonal antibody in cell suspension. The monoclonal antibody can be isolated and purified according to standard methods known to those skilled in the art and described (Bakker et al., 2001, 2006).

### Example 4 Production of galactosylated monoclonal antibody lacking xylose, fucose and high-mannose type N-glycans in alg3 tobacco plant

In a preferred embodiment an alg3-mutant plant such as a Nicotiana tabacum plant described in Example 2, Arabidopsis thaliana alg3-mutant described herein (Example 1), Oryza sativa, Medicago truncatula, M. sativa, Solanum tuberosum, or otherwise obtained through methods described herein, can be provided with a hybrid galactosyltransferase gene, such as a human ß1,4-galactosyltransferase gene of which the CTS-region is exchanged with that of a plant ß1,2-xylosyltransferase gene as decribed in patent application WO03/078637, for the production of a glycoprotein, preferably a recombinant glycoprotein, preferably an antibody. To this end expression cassettes comprising the gene sequences for the respective recombinant glycoprotein(s) and hybrid galactosyltransferase gene need to be be provided under control of appropriate controlling elements such as promoter and terminator sequences and introduced in said alg3-plant by for example but not limited to Agrobacterium-mediated transformation, as known by those skilled in the art. A hybrid human ß1,4-galactosyltransferase gene is for example described in Bakker et al. (2006) and WO03/078637. The resulting transgenic plant can be analysed for the expression of the respective glycoprotein which can be isolated according to methods known to those skilled in the art.
For the production of a recombinant monoclonal antibody, such as MGR48 (NCBI entry AY311598 for light chain and AY311599 for heavy chain sequence) in such a transgenic alg3-tobacco plant expressing the hybrid human galactosyltransferase gene, the alg3-tobacco plant obtained through the method described in Example 2, needs to be re-transformed with a vector, such as a binary vector, comprising expression cassettes for the monoclonal MGR48 antibody genes in conjunction with an expression cassette comprising the hybrid human galactosyltransferase gene, preferable the TmXyl-GalT gene as described in patent application WO03/078637 and described by Bakker et al. (2006). Recombinant monoclonal antibody can be isolated and purified through affinity chromatography as described and analysed for N-glycan composition (Bakker et al., 2001, 2006).

### Example 5 Production of galactosylated glycoprotein lacking xylose, fucose and high-mannose type N-glycans in alg3 plant

In another embodiment an alg3-mutant plant as described herein is provided with a so-called "short-ß1,4-galactosyltransferase gene" as provided in patent application US 2005/0143564 together with an expression cassette providing the production of the recombinant glycoprotein, such as but not limited to a monoclonal antibody.

### Example 6 Production of recombinant gonadotrophin lacking xylose, fucose and high-mannose type N-glycans in plant endoplasmatic reticulum derived protein bodies

In another embodiment an alg3-mutant plant as described herein can be used for the production of recombinant glycoprotein(s) such as but not limited to monoclonal antibodies and glycoprotein hormones, such as but not limited to gonadotrophins, made in plants by accumulation in plant endosplasmatic reticulum-derived protein bodies such as but not limited to those described in patent application WO04/003207. To this end the glycoprotein sequence needs to be provided with a nucleic acid sequence encoding a Y-zein protein, or a fragment thereof capable of directing the glycoprotein towards endoplasmatic reticulum-derived protein bodies resulting in a protein-body-derived recombinant glycoprotein lacking high mannose-type N-glycans typically referred to as Man6GlcNAc2, Man7GlcNAc2, Man8GlcNAc2 or Man9GlcNAc2 and with preferred structures such as Man3GlcNAc2, Man4GrlcNAc2 and to a lesser extent Man5GlcNAc2.
For the production of a gonadotrophin such as human chorionic gonadotropin beta 3 subunit precursor (NCBI entry number NP_000728) in endoplasmatic reticulum derived protein bodies of an alg3-tobacco plant as described in Example 2, the coding sequence of the human chorionic gonadotrophin beta 3 subunit needs to be fused to a Y-zein derived sequence, such as RX3 described in patent application WO04/003207 complemented with a signal peptide sequence for endoplasmatic reticulum import and resulting in a RX3-gonadotrophin fusion protein. The resulting sequence needs to be placed under control of proper controlling elements such as promoter and terminator sequences and placed in a transformation vector for introduction into alg3-tobacco plant. The resulting recombinant gonadotrophin accumulating in endoplasmatic reticulum-derived protein bodies can be isolated by first isolating the protein bodies and successively disrupting or extracting said protein bodies as described in patent application WO04/003207. The N-glycan composition can be analysed using MALDI-TOF as described (Bakker et al., 2006).

### Example 7 Production of recombinant glycoprotein lacking high-mannose type N-glycans in endoplasmatic reticulum derived protein bodies

In another embodiment an alg3-mutant, whether a mammalian cell line (CHO, Per.C6, HEK 293, BHK or otherwise), a yeast cell line (such as Saccharomyces cerevisiae, Pichia pastoris or otherwise), fungus, insect cell line or other organism comprising a N-glycosylation machinery for the production of glycoproteins, can be used for the accumulation of a recombinant glycoprotein in an endoplasmatic reticulum-derived protein body such as described in patent application WO04/003207. To this end an alg3 mutant cell line needs to be constructed similar but not limited to antisense or RNAi technology or transposon mutagenesis and provided with a gene sequence coding for the respective glycoprotein provided with a Y-zein derived sequence, such as but not limited to RX3 and directing the accumulation of the resulting fusion protein to endoplasmatic reticulum-derived protein body. The resulting glycoprotein can be isolated according to technologies known by those skilled in the art.

### References;

Aebi, M., Gassenhuber, J., Domdey, H., and te Heesen, S. (1996). Cloning and characterization of the ALG3 gene of Saccharomyces cerevisiae. Glycobiology 6, 439-444.
Aker, J., Borst, J.W., Karlova, R., and de Vries, S. (2006). The Arabidopsis thaliana AAA protein CDC48A interacts in vivo with the somatic embryogenesis receptor-like kinase 1 receptor at the plasma membrane. J Struct Biol 156, 62-71.
Bakker, H., Bardor, M., Molthoff, J.W. Gomord, V., Elbers, I., Stevens, L.H., Jordi, W., Lommen, A., Faye, L., Lerouge, P., and Bosch, D. (2001) Galactose-extended glycans of antibodies produced by transgenic plants Proc Natl Acad Sci U.S.A. 98(5), 2899-2904.
Bakker H, Rouwendal GJ, Karnoup AS, Florack DE, Stoopen GM, Helsper JP, van Ree R, van Die I, Bosch D. (2006) An antibody produced in tobacco expressing a hybrid beta-1,4-galactosyltransferase is essentially devoid of plant carbohydrate epitopes. Proc Natl Acad Sci U.S.A. 103(20), 7577-82.
Benghezal, M., Wasteneys, G.O., and Jones, D.A. (2000). The C-terminal dilysine motif confers endoplasmic reticulum localization to type I membrane proteins in plants. Plant Cell 12, 1179-1201.
Boisson, M., Gomord, V., Audran, C., Berger, N., Dubreucq, B., Granier, F., Lerouge, P., Faye, L., Caboche, M., and Lepiniec, L. (2001). Arabidopsis glucosidase I mutants reveal a critical role of N-glycan trimming in seed development. Embo J 20, 1010-1019.
Burn, J.E., Hurley, U.A., Birch, R.J., Arioli, T., Cork, A., and Williamson, R.E. (2002). The cellulose-deficient Arabidopsis mutant rsw3 is defective in a gene encoding a putative glucosidase II, an enzyme processing N-glycans during ER quality control. Plant J 32, 949-960.
Davidson, R.C., Nett, J.H., Renfer, E., Li, H., Stadheim, T.A., Miller, B.J., Miele, R.G., Hamilton, S.R., Choi, B.K., Mitchell, T.I., and Wildt, S. (2004). Functional analysis of the ALG3 gene encoding the Dol-P-Man: Man5GlcNAc2-PP-Dol mannosyltransferase enzyme of P. pastoris. Glycobiology 14, 399-407.
Denecke, J., Kranz, C., Kemming, D., Koch, H.G., and Marquardt, T. (2004). An activated 5' cryptic splice site in the human ALG3 gene generates a premature termination codon insensitive to nonsense-mediated mRNA decay in a new case of congenital disorder of glycosylation type Id (CDG-Id). Hum Mutat 23, 477-486.
Denecke, J., Kranz, C., von Kleist-Retzow, J., Bosse, K., Herkenrath, P., Debus, O., Harms, E., and Marquardt, T. (2005). Congenital disorder of glycosylation type Id: clinical phenotype, molecular analysis, prenatal diagnosis, and glycosylation of fetal proteins. Pediatr Res 58, 248-253.
Elbers, I.J., Stoopen, G.M., Bakker, H., Stevens, L.H., Bardor, M., Molthoff, J.W., Jordi, W.J., Bosch, D., and Lommen, A. (2001). Influence of growth conditions and developmental stage on N-glycan heterogeneity of transgenic immunoglobulin G and endogenous proteins in tobacco leaves. Plant Physiol 126, 1314-1322.
Ermonval, M., Cacan, R., Gorgas, K., Haas, I.G., Verbert, A., and Buttin, G. (1997). Differential fate of glycoproteins carrying a monoglucosylated form of truncated N-glycan in a new CHO line, MadIA214214, selected for a thermosensitive secretory defect. J Cell Sci 110 ( Pt 3), 323-336.
Foulquier, F., Harduin-Lepers, A., Duvet, S., Marchal, I., Mir, A.M., Delannoy, P., Chirat, F., and Cacan, R. (2002). The unfolded protein response in a dolichyl phosphate mannose-deficient Chinese hamster ovary cell line points out the key role of a demannosylation step in the quality-control mechanism of N-glycoproteins. Biochem J 362, 491-498.
Gallois, P., Makishima, T., Hecht, V., Despres, B., Laudie, M., Nishimoto, T., and Cooke, R. (1997). An Arabidopsis thaliana cDNA complementing a hamster apoptosis suppressor mutant. Plant J 11, 1325-1331.
Gillmor, C.S., Poindexter, P., Lorieau, J., Palcic, M.M., and Somerville, C. (2002). Alpha-glucosidase I is required for cellulose biosynthesis and morphogenesis in Arabidopsis. J Cell Biol 156, 1003-1013.
Giritch A, Marillonnet S, Engler C, van Eldik G, Botterman J, Klimyuk V, and Gleba Y. (2006). Rapid high-yield expression of full-size IgG antibodies in plants coinfected with noncompeting viral vectors. Proc Natl Acad Sci U S A 103:14645-6, 2006.
Helenius, J., and Aebi, M. (2002). Transmembrane movement of dolichol linked carbohydrates during N-glycoprotein biosynthesis in the endoplasmic reticulum. Semin Cell Dev Biol 13, 171-178.
Huffaker, T.C., and Robbins, P.W. (1982). Temperature-sensitive yeast mutants deficient in asparagine-linked glycosylation. J Biol Chem 257, 3203-3210.
Huffaker, T.C., and Robbins, P.W. (1983). Yeast mutants deficient in protein glycosylation. Proc Natl Acad Sci U S A 80, 7466-7470.
Knauer, R., and Lehle, L. (1999). The oligosaccharyltransferase complex from Saccharomyces cerevisiae. Isolation of the OST6 gene, its synthetic interaction with OST3, and analysis of the native complex. J Biol Chem 274, 17249-17256.
Koiwa, H., Li, F., McCully, M.G., Mendoza, I., Koizumi, N., Manabe, Y., Nakagawa, Y., Zhu, J., Rus, A., Pardo, J.M., Bressan, R.A., and Hasegawa, P.M. (2003). The STT3a subunit isoform of the Arabidopsis oligosaccharyltransferase controls adaptive responses to salt/osmotie stress. Plant Cell 15, 2273-2284.
Korner, C., Knauer, R., Stephani, U., Marquardt, T., Lehle, L., and von Figura, K. (1999); Carbohydrate deficient glycoprotein syndrome type IV: deficiency of dolichyl-P-Man:Man(5)GlcNAc(2)-PP-dolichyl mannosyltransferase. Embo J 18, 6816-6822.
Kornfeld, R., and Kornfeld,' S. (1985). Assembly of asparagine-linked oligosaccharides. Annu Rev Biochem 54, 631-664.
Lerouge, P., Cabanes-Macheteau, M., Rayon, C., Fischette-Laine, A.C., Gomord, V., and Faye, L. (1998). N-glycoprotein biosynthesis in plants: recent developments and future trends. Plant Mol Biol 38, 31-48.
Lerouxel, O., Mouille, G., Andeme-Onzighi, C., Bruyant, M.P., Seveno, M., Loutelier-Bourhis, C., Driouich, A., Hofte, H., and Lerouge, P. (2005). Mutants in DEFECTIVE GLYCOSYLATION, an Arabidopsis homolog of an oligosaccharyltransferase complex subunit, show protein underglycosylation and defects in cell differentiation and growth. Plant J 42, 455-468.
Murashige, T., Skoog, F. (1962) A revised medium for rapid growth and bioassays with tobacco tissue cultures. Physiologia Plantarum 15, 473-497.
Petruccelli S, Otegui MS, Lareu F, Tran Dinh O, Fitchette AC, Circosta A, Rumbo M, Bardor M, Carcamo R, Gomord V, Beachy RN. A KDEL-tagged monoclonal antibody is efficiently retained in the endoplasmic reticulum in leaves, but is both partially secreted and sorted to protein storage vacuoles in seeds. Plant Biotechnol J. 2006 Sep;4(5):511-
Pichler, H., Gaigg, B., Hrastnik, C., Achleitner, G., Kohlwein, S.D., Zellnig, G., Perktold, A., and Daum, G. (2001). A subfraction of the yeast endoplasmic reticulum associates with the plasma membrane and has a high capacity to synthesize lipids. Eur J Biochem 268, 2351-2361.
Roth, J., Ziak, M., and Zuber, C. (2003). The role of glucosidase II and endomannosidase in glucose trimming of asparagine-linked oligosaccharides. Biochimie 85, 287-294.
Runge, K.W., and Robbins, P.W. (1986). A new yeast mutation in the glucosylation steps of the asparagine-linked glycosylation pathway. Formation of a novel asparagine-linked oligosaccharide containing two glucose residues. J Biol Chem 261, 15582-15590.
Runge, K.W., Huffaker, T.C., and Robbins, P.W. (1984). Two yeast mutations in glucosylation steps of the asparagine glycosylation pathway. J Biol Chem 259, 412-417.
Schollen, E., Grunewald, S., Keldermans, L., Albrecht, B., Korner, C., and Matthijs, G. (2005). CDG-Id caused by homozygosity for an ALG3 mutation due to segmental maternal isodisomy UPD3(q21.3-qter). Eur J Med Genet 48, 153-158.
Snider, M.D., Sultzman, L.A., and Robbins, P.W. (1980). Transmembrane location of oligosaccharide-lipid synthesis in microsomal vesicles. Cell 21, 385-392.
Stagljar, I., te Heesen, S., and Aebi, M. (1994). New phenotype of mutations deficient in glucosylation of the lipid-linked oligosaccharide: cloning of the ALG8 locus. Proc Natl Acad Sci U S A 91, 5977-5981.
Stibler, H., Stephani, U., and Kutsch, U. (1995). Carbohydrate-deficient glycoprotein syndrome--a fourth subtype. Neuropediatrics 26, 235-237.
Sturm, V., Kober, B., Hover, K.H., Schlegel, W., Boesecke, R., Pastyr, O., Hartmann, G.H., Schabbert, S., zum Winkel, K., Kunze, S., and et al. (1987). Stereotactic percutaneous single dose irradiation of brain metastases with a linear accelerator. Int J Radiat Oncol Biol Phys 13, 279-282.
Sun, L., Eklund, E.A., Chung, W.K., Wang, C., Cohen, J., and Freeze, H.H. (2005). Congenital disorder of glycosylation id presenting with hyperinsulinemic hypoglycemia and islet cell hyperplasia. J Clin Endocrinol Metab 90, 4371-4375.
Verostek, M.F., Atkinson, P.H., and Trimble, R.B. (1993). Glycoprotein biosynthesis in the alg3 Saccharomyces cerevisiae mutant. I. Role of glucose in the initial glycosylation of invertase in the endoplasmic reticulum. J Biol Chem 268, 12095-12103.
Vitale, A., and Chrispeels, M.J. (1984). Transient N-acetylglucosamine in the biosynthesis of phytohemagglutinin: attachment in the Golgi apparatus and removal in protein bodies. J Cell Biol 99, 133-140.
Zufferey, R., Knauer, R., Burda, P., Stagljar, I., te Heesen, S., Lehle, L., and Aebi, M. (1995). STT3, a highly conserved protein required for yeast oligosaccharyl transferase activity in vivo. Embo J 14, 4949-4960.

## Claims

1. A method to modify glycosylation in plant cells or plants by partially inhibiting Alg3 expression.

2. A method according to claim 1, wherein said modification concerns N-glycan biosynthesis.

3. A method according to claim 1 and 2, wherein said modification comprises a reduction in high mannose type glycans.

4. A method according to any of claims 1-3, wherein a mutant Alg3 gene is introduced in the plant cell.

5. A method according to claim 4, wherein the Alg3 gene is an *Arabidopsis* Alg3 gene.

6. A method according to any of claims 5 or 6, wherein the mutant Alg3 gene has a DNA insert between intron 11 and exon 12.

7. A method according to claim 6, wherein said mutant Alg3 gene is derived from the *Arabidopsis* plant line Salk_040296c.

8. A splice mutant of the Arabidopsis Alg3 gene, **characterised in that** it has a DNA insert between intron 11 and exon 12.

9. A splice mutant according to claim 9, **characterised in that** it is derived from the *Arabidopsis* plant line Salk_040296c.

10. Use of a splice mutant according to claims 8 or 9 for the reduction of high mannose type glycans in plant cells or plants.

11. Transgenic plant cell or plant in which the expression of Alg3 is partially inhibited, wherein said plant is not plant line Salk_064006 or Salk_040296c.

12. Transgenic plant according to claim 11, which comprises a splice mutant according to any of claims 9-11.

13. Transgenic plant according to claim 11, which comprises an antisense Alg3 construct, optionally under control of a tissue-specific promoter.

14. Transgenic plant according to claim 11, which comprises an Alg3 dsRNAi construct under control of a tissue specific promoter.

15. Transgenic plant according to claim 11, which comprises a co-sense Alg3 construct, optionally under control of a tissue-specific promoter.

16. Transgenic plant according to any of claims 13-15, in which the tissue-specific promoter is able to drive expression in tissues selected from the group consisting of storage organs (such as tubers or seeds), leaves and roots.

17. Method to produce proteins reduced in high mannose type glycans in plants by culturing a transgenic plant according to any of claims 11-16, optionally providing said plant with a nucleic acid encoding a protein of interest, and harvesting the glycosylated proteins.


SEQUENCE LISTING

<110>  Plant Research International B.V.

<120>  Alg3 mutant

<130>  P80679EP00

<140>  EP 07108728.2
<141>  2007-05-23

<160>  25

<170>  PatentIn version 3.3

<210>  1
<211>  438
<212>  PRT
<213>  Homo sapiens

<400>  1

<210>  2
<211>  458

<212>  PRT
<213>  Saccharomyces cerevisiae

<400>  2

Met Glu Gly Glu Gln Ser Pro Gln Gly Glu Lys Ser Leu Gln Arg Lys
1               5                   10                  15

Gln Phe Val Arg Pro Pro Leu Asp Leu Trp Gln Asp Leu Lys Asp Gly
            20                  25                  30

Val Arg Tyr Val Ile Phe Asp Cys Arg Ala Asn Leu Ile Val Met Pro
        35                  40                  45

Leu Leu Ile Leu Phe Glu Ser Met Leu Cys Lys Ile Ile Ile Lys Lys
    50                  55                  60

Val Ala Tyr Thr Glu Ile Asp Tyr Lys Ala Tyr Met Glu Gln Ile Glu
65                  70                  75                  80

Met Ile Gln Leu Asp Gly Met Leu Asp Tyr Ser Gln Val Ser Gly Gly
                85                  90                  95

Thr Gly Pro Leu Val Tyr Pro Ala Gly His Val Leu Ile Tyr Lys Met
            100                 105                 110

Met Tyr Trp Leu Thr Glu Gly Met Asp His Val Glu Arg Gly Gln Val
        115                 120                 125

Phe Phe Arg Tyr Leu Tyr Leu Leu Thr Leu Ala Leu Gln Met Ala Cys
    130                 135                 140

Tyr Tyr Leu Leu His Leu Pro Pro Trp Cys Val Val Leu Ala Cys Leu
145                 150                 155                 160

Ser Lys Arg Leu His Ser Ile Tyr Val Leu Arg Leu Phe Asn Asp Cys
                165                 170                 175

Phe Thr Thr Leu Phe Met Val Val Thr Val Leu Gly Ala Ile Val Ala
            180                 185                 190

Ser Arg Cys His Gln Arg Pro Lys Leu Lys Lys Ser Leu Ala Leu Val
        195                 200                 205

Ile Ser Ala Thr Tyr Ser Met Ala Val Ser Ile Lys Met Asn Ala Leu
    210                 215                 220

Leu Tyr Phe Pro Ala Met Met Ile Ser Leu Phe Ile Leu Asn Asp Ala
225                 230                 235                 240

Asn Val Ile Leu Thr Leu Leu Asp Leu Val Ala Met Ile Ala Trp Gln
                245                 250                 255

Val Ala Val Ala Val Pro Phe Leu Arg Ser Phe Pro Gln Gln Tyr Leu
            260                 265                 270

His Cys Ala Phe Asn Phe Gly Arg Lys Phe Met Tyr Gln Trp Ser Ile
        275                 280                 285

Asn Trp Gln Met Met Asp Glu Glu Ala Phe Asn Asp Lys Arg Phe His
    290                 295                 300

Leu Ala Leu Leu Ile Ser His Leu Ile Ala Leu Thr Thr Leu Phe Val
305                 310                 315                 320

Thr Arg Tyr Pro Arg Ile Leu Pro Asp Leu Trp Ser Ser Leu Cys His
                325                 330                 335

Pro Leu Arg Lys Asn Ala Val Leu Asn Ala Asn Pro Ala Lys Thr Ile
            340                 345                 350

Pro Phe Val Leu Ile Ala Ser Asn Phe Ile Gly Val Leu Phe Ser Arg
        355                 360                 365

Ser Leu His Tyr Gln Phe Leu Ser Trp Tyr His Trp Thr Leu Pro Ile
    370                 375                 380

Leu Ile Phe Trp Ser Gly Met Pro Phe Phe Val Gly Pro Ile Trp Tyr
385                 390                 395                 400

Val Leu His Glu Trp Cys Trp Asn Ser Tyr Pro Pro Asn Ser Gln Ala
                405                 410                 415

Ser Thr Leu Leu Leu Ala Leu Asn Thr Val Leu Leu Leu Leu Leu Ala
            420                 425                 430

Leu Thr Gln Leu Ser Gly Ser Val Ala Leu Ala Lys Ser His Leu Arg
        435                 440                 445

Thr Thr Ser Ser Met Glu Lys Lys Leu Asn
    450                 455

<210> 3
<211> 438
<212> PRT
<213> Arabidopsis thaliana

<400> 3

Met Ala Gly Ala Ser Ser Pro Ala Ser Leu Arg Ala Ser Arg Ser Arg
1               5                   10                  15

Arg Leu Gly Lys Glu Thr Asn Arg Ser Asp Leu Phe Lys Lys Pro Ala
            20                  25                  30

Val Pro Phe Ala Phe Ala Leu Ile Leu Ala Asp Ala Ile Leu Val Ala
        35                  40                  45

Leu Ile Ile Ala Tyr Val Pro Tyr Thr Lys Ile Asp Trp Asp Ala Tyr
    50                  55                  60

Met Ser Gln Val Ser Gly Phe Leu Gly Gly Glu Arg Asp Tyr Gly Asn
65                  70                  75                  80

Leu Lys Gly Asp Thr Gly Pro Leu Val Tyr Pro Ala Gly Phe Leu Tyr
                85                  90                  95

Val Tyr Ser Ala Val Gln Asn Leu Thr Gly Gly Glu Val Tyr Pro Ala
            100                 105                 110

Gln Ile Leu Phe Gly Val Leu Tyr Ile Val Asn Leu Gly Ile Val Leu
        115                 120                 125

Ile Ile Tyr Val Lys Thr Asp Val Val Pro Trp Trp Ala Leu Ser Leu
    130                 135                 140

Leu Cys Leu Ser Lys Arg Ile His Ser Ile Phe Val Leu Arg Leu Phe
145                 150                 155                 160

Asn Asp Cys Phe Ala Met Thr Leu Leu His Ala Ser Met Ala Leu Phe
                165                 170                 175

Leu Tyr Arg Lys Trp His Leu Gly Met Leu Val Phe Ser Gly Ala Val
            180                 185                 190

Ser Val Lys Met Asn Val Leu Leu Tyr Ala Pro Thr Leu Leu Leu Leu
        195                 200                 205

Leu Leu Lys Ala Met Asn Ile Ile Gly Val Val Ser Ala Leu Ala Gly
    210                 215                 220

Ala Ala Leu Val Gln Ile Leu Val Gly Leu Pro Phe Leu Ile Thr Tyr
225                 230                 235                 240

Pro Val Ser Tyr Ile Ala Asn Ala Phe Asp Leu Gly Arg Val Phe Ile
                245                 250                 255

His Phe Trp Ser Val Asn Phe Lys Phe Val Pro Glu Arg Val Phe Val
            260                 265                 270

Ser Lys Glu Phe Ala Val Cys Leu Leu Ile Ala His Leu Phe Leu Leu
        275                 280                 285

Val Ala Phe Ala Asn Tyr Lys Trp Cys Lys His Glu Gly Gly Ile Ile
    290                 295                 300

Gly Phe Met Arg Ser Arg His Phe Phe Leu Thr Leu Pro Ser Ser Leu
305                 310                 315                 320

Ser Phe Ser Asp Val Ser Ala Ser Arg Ile Ile Thr Lys Glu His Val
                325                 330                 335

Val Thr Ala Met Phe Val Gly Asn Phe Ile Gly Ile Val Phe Ala Arg
            340                 345                 350

Ser Leu His Tyr Gln Phe Tyr Ser Trp Tyr Phe Tyr Ser Leu Pro Tyr
        355                 360                 365

Leu Leu Trp Arg Thr Pro Phe Pro Thr Trp Leu Arg Leu Ile Met Phe
    370                 375                 380

Leu Gly Ile Glu Leu Cys Trp Asn Val Tyr Pro Ser Thr Pro Ser Ser
385                 390                 395                 400

Ser Gly Leu Leu Leu Cys Leu His Leu Ile Ile Leu Val Gly Leu Trp
                405                 410                 415

Leu Ala Pro Ser Val Asp Pro Tyr Gln Leu Lys Glu His Pro Lys Ser
            420                 425                 430

Gln Ile His Lys Lys Ala
        435

<210>  4
<211>  26
<212>  DNA
<213>  Artificial

<220>
<223>  Alg3 F2.2 primer

<400>  4
cgttcagtga tgtatcagcc tcacgg                                          26

<210>  5
<211>  21
<212>  DNA
<213>  Artificial

<220>
<223>  Alg3-F3 primer

<400>  5
aagcgttgtc catttacgat g                                               21

<210>  6
<211>  30
<212>  DNA
<213>  Artificial

<220>
<223>  Alg3-R2 primer

<400>  6
ggatttaggg tgttctttga gctgataagg                                        30


<210>  7
<211>  21
<212>  DNA
<213>  Artificial

<220>
<223>  Alg3-R3 primer

<400>  7
cttataattt gcgaacgcca c                                                 21


<210>  8
<211>  23
<212>  DNA
<213>  Artificial

<220>
<223>  actin primer 1

<400>  8
ggtaacattg tgctcagtgg tgg                                               23


<210>  9
<211>  23
<212>  DNA
<213>  Artificial

<220>
<223>  actin primer 2

<400>  9
aacgacctta atcttcatgc tgc                                               23


<210>  10
<211>  25
<212>  DNA
<213>  Artificial

<220>
<223>  AtUTR2 primer 1

<400>  10
cacatttatc ggtcaagtct ccgtt                                             25


<210>  11
<211>  24
<212>  DNA
<213>  Artificial

<220>
<223>  AtUTR2 primer 2

<400>  11
tcgcaggagg cgatggtgat agag                                              24


<210>  12
<211>  23
<212>  DNA
<213>  Artificial

<220>
<223>  T upstream primer 1

<400>  12
cgcacttatt atcgcctatg ttc                                              23


<210>  13
<211>  20
<212>  DNA
<213>  Artificial

<220>
<223>  T upstream primer 2

<400>  13
gccctgtatc gcctttcaag                                                  20


<210>  14
<211>  24
<212>  DNA
<213>  Artificial

<220>
<223>  T downstream primer 1

<400>  14
acttctattc gctaccttat ctac                                             24


<210>  15
<211>  18
<212>  DNA
<213>  Artificial

<220>
<223>  T downstream primer 2

<400>  15
ggaggacggt gttgatgg                                                    18


<210>  16
<211>  38
<212>  DNA
<213>  Artificial

<220>
<223>  ubiquitin-10 primer 1

<400>  16
gtgacgagct cgtaaatttc tgtgttcctt attctctc                              38


<210>  17
<211>  39
<212>  DNA
<213>  Artificial

<220>
<223>  ubiquitin-10 primer 2

<400>  17
gtgacaagct tctgttaatc agaaaaactc agattaatc                             39

<210> 18
<211> 32
<212> DNA
<213> Artificial

<220>
<223> tobacco alg3 primer 1

<400> 18
gtgaccatgg atgcttatat gtctcaggtt ac 32


<210> 19
<211> 33
<212> DNA
<213> Artificial

<220>
<223> tobacco alg3 primer 2

<400> 19
gtgacgagct cagaagtgga tgaaaacacg acc 33


<210> 20
<211> 33
<212> DNA
<213> Artificial

<220>
<223> tobacco alg3 primer 3

<400> 20
gtgacggatc catgcttata tgtctcaggt tac 33


<210> 21
<211> 30
<212> DNA
<213> Artificial

<220>
<223> tobacco alg3 primer 4

<400> 21
gtgactctag aagtggatga aaacacgacc 30


<210> 22
<211> 304
<212> DNA
<213> Arabidopsis thaliana

<400> 22
gtaaatttct gtgttcctta ttctctcaaa atcttcgatt ttgttttcgt tcgatcccaa 60

tttcgtatat gttctttggt ttagattctg ttaatcttag atcgaagacg attttctggg 120

tttgatcgtt agatatcatc ttaattctcg attagggttt catagatatc atccgatttg 180

ttcaaataat ttgagttttg tcgaataatt actcttcgat ttgtgatttc tatctagatc 240

tggtgttagt ttctagtttg tgcgatcgaa tttgtcgatt aatctgagtt tttctgatta 300

acag 304


<210> 23

<211>  121
<212>  DNA
<213>  Artificial

<220>
<223>  CASesp

<400>  23
ggcgcgcctc gaggcgatcg cagatctatc gatgcatgcc atggtacccg ggagctcgaa      60

ttctagaagc ttctgcagac gcgtcgacgt catatggatc cgcgagagac gtcttaatta     120

a                                                                     121


<210>  24
<211>  1229
<212>  DNA
<213>  Artificial

<220>
<223>  RAP40

<400>  24
ggcgcgccaa gcttgaatta attctactcc aaaaatatca aagatacagt ctcagaagac      60

caaagggcaa ttgagacttt tcaacaaagg gtaatatccg gaaacctcct cggattccat     120

tgcccagcta tctgtcactt tattgtgaag atagtggaaa aggaaggtgg ctcctacaaa     180

tgccatcatt gcgataaagg aaaggccatc gttgaagatg cctctgccga cagtggtccc     240

aaagatggac ccccacccac gaggagcatc gtggaaaaag aagacgttcc aaccacgtct     300

tcaaagcaag tggattgatg tgataattcc gcatggagtc aaagattcaa atagaggacc     360

taacagaact cgccgtaaag actggcgaac agttcataca gagtctctta cgactcaatg     420

acaagaagaa aatcttcgtc aacatggtgg agcacgacac acttgtctac tccaaaaata     480

tcaaagatac agtctcagaa gaccaaaggg caattgagac ttttcaacaa agggtaatat     540

ccggaaacct cctcggattc cattgcccag ctatctgtca ctttattgtg aagatagtgg     600

aaaaggaagg tggctcctac aaatgccatc attgcgataa aggaaaggcc atcgttgaag     660

atgcctctgc cgacagtggt cccaaagatg gacccccacc cacgaggagc atcgtggaaa     720

aagaagacgt tccaaccacg tcttcaaagc aagtggattg atgtgatatc tccactgacg     780

taagggatga cgcacaatcc cactatcctt cgcaagaccc ttcctctata taaggaagtt     840

catttcattt ggagagggtt tttattttta attttctttc aaatacttcc accatgggcg     900

agctcggtac ccggggatcc ccgggtaccg agctcgaatt tccccgatcg ttcaaacatt     960

tggcaataaa gtttcttaag attgaatcct gttgccggtc ttgcgatgat tatcatataa    1020

tttctgttga attacgttaa gcatgtaata attaacatgt aatgcatgac gttatttatg    1080

agatgggttt ttatgattag agtcccgcaa ttatacattt aatacgcgat agaaaacaaa    1140

atatagcgcg caaactagga taaattatcg cgcgcggtgt catctatgtt actagatcgg    1200

gaattccaga tctgcggccg cttaattaa                                      1229


<210>  25

<211>  593
<212>  DNA
<213>  Nicotiana tabacum

<400>  25
ggatgcttat atgtctcagg ttactggctt tcttgaagga gagagggatt atagtaactt      60

gaaaggtgat acagggcctc tagtttatcc agcaggcttt ctttatattt actctgctat     120

tcaatacgtt accggaggtc aagtctatcc tgctcagatt ctttttggct ttctctacat     180

gctggatcta gcaattgtcg tgttcatcta cttgaagact gatgtggtac cttggtgggc     240

tctttccttg ctttctctgt ctaaaagagt tcactctatc tttgttcttc gattatttaa     300

tgattgttta gccactactc tcctccatgc tgcattgatc tcaattattt atcaaaaatg     360

gcatctaggg ttggtaattt tcagtggagc tgtttctata aagatgaatg tgctcctgta     420

tgcaccacct ctgttgctcc tgatggtgaa ggcaatggat attgttggag ttatatctgc     480

tttagcaggt gctgcattag tgcagattgt cattgggctg ccttttgtcc tgtcacatcc     540

agcttcatat atatcaaaag ctttcgatct tggtcgtgtt ttcatccact tct            593